(19) Europäisches Patentamt
European Patent Office
Office européen des brevets

(11) **EP 4 200 856 B1**

(12) **EUROPEAN PATENT SPECIFICATION**

(45) Date of publication and mention
of the grant of the patent:
**18.10.2023 Bulletin 2023/42**

(21) Application number: **22789995.2**

(22) Date of filing: **05.10.2022**

(51) International Patent Classification (IPC):
***G16B 20/00*** *(2019.01)*

(52) Cooperative Patent Classification (CPC):
**G16H 50/30; G16B 20/20; G16H 10/20;
G16H 20/10; G16H 50/70;** G06N 20/10

(86) International application number:
**PCT/GB2022/052515**

(87) International publication number:
**WO 2023/062339 (20.04.2023 Gazette 2023/16)**

(54) **COMPUTER-IMPLEMENTED METHOD AND APPARATUS FOR ANALYSING GENETIC DATA**

COMPUTERIMPLEMENTIERTES VERFAHREN UND VORRICHTUNG ZUR ANALYSE
GENETISCHER DATEN

PROCÉDÉ MIS EN OEUVRE PAR ORDINATEUR ET APPAREIL POUR L'ANALYSE DE DONNÉES
GÉNÉTIQUES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **12.10.2021 GB 202114554**

(43) Date of publication of application:
**28.06.2023 Bulletin 2023/26**

(73) Proprietor: **Genomics Plc**
**Oxford, Oxfordshire OX1 1JD (GB)**

(72) Inventors:
• **WEALE, Michael**
**Oxford OX1 1JD (GB)**
• **PLAGNOL, Vincent Yann Marie**
**Oxford OX1 1JD (GB)**
• **MOORE, Rachel**
**Oxford OX1 1JD (GB)**
• **WELLS, Daniel**
**Oxford OX1 1JD (GB)**
• **SETH, Priyanka**
**Oxford OX1 1JD (GB)**
• **PALMER, Duncan**
**Oxford OX1 1JD (GB)**

(74) Representative: **J A Kemp LLP**
**80 Turnmill Street**
**London EC1M 5QU (GB)**

(56) References cited:
• **WANG MINXIAN ET AL: "Validation of a
Genome-Wide Polygenic Score for Coronary
Artery Disease in South Asians", JOURNAL OF
THE AMERICAN COLLEGE OF CARDIOLOGY,
ELSEVIER, AMSTERDAM, NL, vol. 76, no. 6, 3
August 2020 (2020-08-03) , pages 703-714,
XP086236723, ISSN: 0735-1097, DOI:
10.1016/J.JACC.2020.06.024 [retrieved on
2020-08-03]**
• **Privé Florian ET AL: "High-resolution portability
of 245 polygenic scores when derived and
applied in the same cohort", medRxiv, 23
February 2021 (2021-02-23), pages 1-20,
XP093003158, DOI: 10.1101/2021.02.05.21251061
Retrieved from the Internet:
URL:https://www.medrxiv.org/content/10.110
1/2021.02.05.21251061v2.full.pdf+html [retrieved
on 2022-11-29] cited in the application**
• **BITARELLO BÁRBARA D ET AL: "Polygenic
Scores for Height in Admixed Populations", G3
GENES|GENOMES|GENETICS, vol. 10, no. 11, 1
November 2020 (2020-11-01), pages 4027-4036,
XP093002681, ISSN: 2160-1836, DOI:
10.1534/g3.120.401658 cited in the application**

EP 4 200 856 B1

- Fritscheid Lars G ET AL: "On cross-ancestry cancer polygenic risk scores", PLOS GENETICS, 16 September 2021 (2021-09-16), pages 1-14, XP093002690, DOI: https://doi.org/10.1371/journal.pgen.10096 70 Retrieved from the Internet: URL:https://journals.plos.org/plosgenetics /article?id=10.1371/journal.pgen.1009670 [retrieved on 2022-11-28] cited in the application
- AMARIUTA TIFFANY ET AL: "Improving the trans-ancestry portability of polygenic risk scores by prioritizing variants in predicted cell-type-specific regulatory elements", NATURE GENETICS, vol. 52, no. 12, 30 December 2020 (2020-12-30), pages 1346-1354, XP037309453, ISSN: 1061-4036, DOI: 10.1038/S41588-020-00740-8 cited in the application
- Ruan Yunfeng ET AL: "Improving Polygenic Prediction in Ancestrally Diverse Populations", medRxiv, 24 August 2021 (2021-08-24), XP055893575, DOI: 10.1101/2020.12.27.20248738 Retrieved from the Internet: URL:https://www.medrxiv.org/content/10.110 1/2020.12.27.20248738v2.full.pdf [retrieved on 2022-02-18]
- BODEA CORNELIU A ET AL: "A Method to Exploit the Structure of Genetic Ancestry Space to Enhance Case-Control Studies", THE AMERICAN JOURNAL OF HUMAN GENETICS, AMERICAN SOCIETY OF HUMAN GENETICS , CHICAGO , IL, US, vol. 98, no. 5, 14 April 2016 (2016-04-14) , pages 857-868, XP029530721, ISSN: 0002-9297, DOI: 10.1016/J.AJHG.2016.02.025
- LI JIARUI ET AL: "Robust genome-wide ancestry inference for heterogeneous datasets: illustrated using the 1,000 genome project with 3D facial images", SCIENTIFIC REPORTS, vol. 10, no. 1, 16 July 2020 (2020-07-16), XP093002693, ISSN: 2045-2322, DOI: 10.1038/s41598-020-68259-w
- LAMBERT SAMUEL A ET AL: "The Polygenic Score Catalog as an open database for reproducibility and systematic evaluation", NATURE GENETICS, vol. 53, no. 4, 10 March 2021 (2021-03-10) , pages 420-425, XP037419783, ISSN: 1061-4036, DOI: 10.1038/S41588-021-00783-5

**Description**

The invention relates to analysing genetic and phenotype data about an organism to obtain information about the organism, in particular for enabling improved estimates of the risk of the organism having a target phenotype or target phenotype combination based on an ancestry of the organism.

**Background**

A polygenic risk score (PRS) is a quantitative summary of the contribution of an organism's inherited DNA to the phenotypes that it may exhibit. A PRS may include in its computation all DNA variants relevant (either directly or indirectly) to a phenotype of interest or may use its component parts if these are more relevant to a particular aspect of an organism's biology (including cells, tissues, or other biological units, mechanisms or processes). A PRS can be used directly, or as part of a plurality of measurements or records about the organism, to infer aspects of its past, current, and future biology.

PRSs are gaining traction as a tool for disease prevention, stratification and diagnosis. In the context of improving human health and healthcare, PRSs have a range of practical uses, which include, but are not limited to: predicting the risk of developing a disease or phenotype, predicting age of onset of a phenotype, predicting disease severity, predicting disease subtype, predicting the response to treatment, selecting appropriate screening strategies for an individual, selecting appropriate medication interventions, and setting prior probabilities for other prediction algorithms.

PRS may have direct use as a source of input in the application of artificial intelligence and machine learning approaches to making predictions or classifications from other high dimensional input data (for example imaging). They may be used to help train these algorithms, for example to identify predictive measurements based on non-genetic data. As well as having utility in making predictive statements about an individual, they can also be used to identify cohorts of individuals, included but not limited to the above applications, by calculating the PRS for a large number of individuals, and then grouping individuals on the basis of the PRSs.

PRSs can also aid in the selection of individuals for clinical trials, for example to optimise trial design by recruiting individuals more likely to develop the relevant disease or phenotypes, thereby enhancing the assessment of the efficacy of a new treatment. PRSs carry information about the individuals they are calculated for, but also for their relatives (who share a fraction of their inherited DNA). Information about the impact of an individual's DNA on their phenotypes can derive from any relevant assessment of the potential impact of carrying any particular combination of DNA variants.

In what follows we focus on the analysis of the recent wealth of information that derives from genetic association studies (GAS). These studies systematically assess the potential contribution of DNA variants to the genetic basis of a phenotype. Since the mid-2000s, GAS (typically genome-wide association studies: GWAS, or association studies targeting single variants, or variants in a region of the genome, or GWAS restricted to a particular region of the genome) have been conducted on many thousands of (largely human) phenotypes, in millions of individuals, generating billions of potential links between genotypes and phenotypes. The resulting raw data is often then simplified to produce summary statistic data. GAS summary statistic data consists of, for each genetic variant (whether imputed or observed), the inferred effect size of the genetic variant on the phenotype of the GAS and the standard error of the inferred effect size. In other cases the individual level data, consisting of a full genetic profile of the individuals in a study and information about their phenotypes, may be available directly. However, individual level data is typically less widely available due to requirements on the privacy of an individual's data.

PRSs aggregate a large number of appropriately weighted genetic variants in order to provide individual specific relative risks of a disease, such as coronary artery disease or breast cancer. However, the mapping from weighted genetic variants to relative risk is not straightforward. One reason is the fact that analytical strategies used to generate PRSs typically yield uncalibrated scores, i.e. the PRS is not readily interpretable even in the population it was derived for. Furthermore, the interpretation of the PRS varies across populations, because the effect size associated with each genetic variant varies as a function of ancestry. Hence the aggregated "effect size per unit of PRS" is not constant across human populations.

Given these constraints, one practical option is to test a PRS in a collection of cohorts representing diverse human populations. One can then estimate the effect size of the PRS in each of these cohorts. Minxian Wang et al ("Validation of a Genome-Wide Polygenic Score for Coronary Artery Disease in South Asians", Journal of the American College of Cardiology, vol. 76, no. 6, 3 August 2020, pages 703-714) is an example of this approach for a cohort of South Asian ancestry. However, this is not always possible because the relevant data (combining genetic and outcome data) may not exist, or not all individuals may fit cleanly into well-defined ancestry groups. Mixed ancestry individuals, or individuals originating from small or understudied populations, often do not fit into the small number of commonly used ancestry groups.

The challenge is extrapolation: based on limited case-control or prospective datasets from particular populations, it is necessary to extrapolate the PRS effect size to individuals who either originate from different populations, or who do not exactly fit the features of the training set. Existing methods for calculating PRS and relative risks do not adequately account for these factors, leading to scores that are inaccurate for a large proportion of individuals.

In order to address these and other limitations, according to a first aspect of the invention, there is provided a computer-implemented method of analysing genetic data according to claim 1.

Using the individual's position in an ancestry space to determine the genetic contribution to risk allows the method to account for individuals who do not fit neatly into one of a small number of predetermined ancestries. This can improve risk estimation and the ability to make appropriate interventions based on genetic risk.

The individual position is represented by a combination of continuous or pseudo-continuous variables, or the individual position comprises an assignment to a weighted combination of a plurality of ancestries. Using continuous or pseudo-continuous variables further improves the ability to rank and compare the positions. Using a weighted combination of ancestries allows for better predictions for mixed-ancestry individuals.

The genetic contribution has a continuous or pseudo-continuous dependency on the individual position. Allowing the genetic contribution to vary at least pseudo-continuously with position improves the resolution with which mixed-ancestry individuals can be assigned an appropriate risk.

In some embodiments, the genetic contribution comprises a sum of sub-contributions for each axis of the ancestry space, each sub-contribution calculated using a coordinate of the individual position along the respective axis of the ancestry space. This allows for the greatest flexibility in how the genetic contribution varies with position by taking account of the position relative to each axis of the ancestry space.

In some embodiments, the ancestry space is non-isotropic, such that a dependency of each sub-contribution on the respective coordinate of the individual position differs between the sub-contributions. This further improves the greatest flexibility in how the genetic contribution varies with position in the ancestry space.

In some embodiments, at least two of the sub-contributions have dependencies on the respective coordinates of the individual position that are related by a shared prior. Relating the dependencies in different axes introduces constraints that can reduce a tendency for overfitting when determining the dependencies.

In some embodiments, the shared prior is specified such that the dependencies of the at least two sub-contributions are sampled from the same distribution. This can be advantageous when it is known that the variation in different axes will have a similar functional form, but may vary in its specific value.

In some embodiments, the shared prior is determined using training data from a plurality of training individuals and one or more predetermined hyperparameters. The use of hyperparameters allows for the strength of the constraint on fitting to be varied depending on the information available.

In some embodiments, each sub-contribution comprises a product of the polygenic risk score and the coordinate of the individual position along the respective axis of the ancestry space. This is a straightforward and effective way to combine the PRS and the coordinates in the ancestry space.

In some embodiments, calculating the genetic contribution comprises calculating a distance in the ancestry space between the individual position and a reference position in the ancestry space, and calculating the genetic contribution using the distance. By reducing the variation to depend on a single distance, the risk of extrapolating outside a known region of the ancestry space is reduced.

In some embodiments, the reference position is a position in the ancestry space of an ancestry used to train coefficients used to calculate the polygenic risk score. This is generally an appropriate reference point, as it represents the point where the PRS is likely to have the largest effect size, with a decrease likely in any direction.

In some embodiments, the ancestry space is defined using reference genetic data from a plurality of reference individuals having a plurality of different ancestries; and calculating the genetic contribution comprises scaling each axis of the ancestry space using a variance accounted for by the respective axis in the reference genetic data before calculating the distance. This scaling means that the most significant axes in terms of the variance they explain will contribute most to the distance, thereby weighting them more heavily.

In some embodiments, the distance is a Euclidean distance in the ancestry space. Euclidean distance is a straightforward and easily calculated measure of distance.

In some embodiments, the genetic contribution comprises a product of the polygenic risk score and the distance. This is a straightforward and effective way to combine the PRS and the distance in the ancestry space.

In some embodiments, calculating the genetic contribution comprises using a linear dependency on the individual position. A linear dependency provides a straightforward way to model the dependence with a consistent behaviour.

In some embodiments, calculating the genetic contribution comprises using a non-linear dependency on the individual position. Non-linear dependencies allow for more complex dependencies that may be appropriate in some circumstances.

In some embodiments, the non-linear dependency comprises a regularised function. Using a regularised function ensures that the dependency has a reasonable and smooth form, and helps to avoid overfitting particularly where data is sparse. For example, in some embodiments, the non-linear dependency comprises a penalised B-spline.

In some embodiments, the non-linear dependency is determined using a Gaussian process as a prior distribution for calculating the genetic contribution using Bayesian inference. Gaussian processes are suitable for determining non-linear dependencies, particularly where data is obtained from a stochastic process with an unknown functional dependence, such as for genetics.

In some embodiments, the Gaussian process has a zero mean vector. This simplifies the Gaussian process analysis, and does not affect the generality of the method, as mean effects can be added later.

In some embodiments, the Gaussian process has a mean vector corresponding to a prior estimate of the genetic contribution to the risk for the target individual. This allows the method to control the odds ratio in regions of the ancestry space away from reference genetic data, to account for knowledge about the risk in different populations.

In some embodiments, a kernel function of the Gaussian process is a stationary function. This choice helps to ensure that the variation of the genetic contribution is similar in different parts of the ancestry space, which would generally be expected even if absolute values differ.

In some embodiments, a kernel function of the Gaussian process decays to zero with decreasing similarity between samples. This helps to ensure that the genetic contribution is not distorted in regions of the ancestry space with relatively sparse reference genetic data. For example, in some embodiments, the kernel function is a radial basis function or a rational quadratic covariance function.

In some embodiments, a posterior distribution for the Bayesian inference is determined using the Gaussian process and training data from a plurality of training individuals having a plurality of different ancestries. This links the posterior distribution to the particular dataset in use.

In some embodiments, determining the posterior distribution comprises approximating the posterior distribution as a Gaussian distribution. This helps to maintain tractability, as some implementations can result in a non-Gaussian distribution.

In some embodiments, a kernel function of the Gaussian process depends on one or more hyperparameters. Using hyperparameters to control the kernel function guards against overfitting, and enforces a meaningful concept of distance in the ancestry space. For example, in some embodiments, the hyperparameters comprise a hyperparameter associated with each of the polygenic risk score, the individual position, and an interaction between the polygenic risk score and the position.

In some embodiments, the genetic contribution comprises an ancestry-independent component calculated using the polygenic risk score that is not dependent on the individual position. This allows the PRS to contribute to the risk to a certain degree regardless of the variation due to the individual position in ancestry space.

In some embodiments, the genetic contribution comprises an ancestry-dependent component calculated based on the individual position that is not dependent on the polygenic risk score. This can allow the risk to account for an increased risk due to ancestry, regardless of other individual genetic variation.

The ancestry space is defined using reference genetic data from a plurality of reference individuals having a plurality of different ancestries. In some embodiments, each reference individual is assigned to one of a plurality of ancestries. Using reference genetic data from individuals having different ancestries allows the method to account for a wide range of different ancestries in determining the ancestry space and the individual position.

In some embodiments, a coordinate system of the ancestry space is determined by applying dimension reduction to the reference genetic data. This is an efficient technique for characterising high-dimensional data, such as genetic data, in a more compact and efficient manner. For example, in some embodiments, the dimension reduction comprises principal component analysis or independent component analysis.

In some embodiments, the dimension reduction comprises a discretisation of the ancestry space into a finite set of ancestries, and the individual position comprises a continuous or pseudo-continuous membership proportion for each ancestry of the finite set of ancestries. This provides a straightforward way to express the individual position in terms of the defined ancestry groups.

In some embodiments, the coordinate system of the ancestry space is chosen to maximise the variance in the reference genetic data accounted for by the ancestry space. This ensures that the genetic contribution will account as best as possible for variation due to ancestry.

In some embodiments, the ancestry space has a dimensionality lower than that of the individual genetic data, and determining the individual position comprises projecting the individual genetic data onto the ancestry space. This allows the genetic contribution for a new individual to be calculated.

In some embodiments, a dependency of the genetic contribution on the individual position and the polygenic risk score is determined using training data from a plurality of training individuals having a plurality of different ancestries, the training data comprising, for each of the training individuals, genetic data, and whether the training individual has the target phenotype or phenotype combination. The training data may or not be the same as the reference genetic data, depending on the specific information available from different studies.

In some embodiments, the training data further comprises, for each of the training individuals, data informative about one or more non-genetic covariates, and the genetic contribution is jointly estimated in the presence of the non-genetic covariates; and the method further comprises receiving individual covariate data for the target individual, the individual covariate data informative about the additional non-genetic covariates for the target individual. This allows the genetic contribution to take account of other factors that may indirectly influence the genetic contribution, and may themselves be correlated to ancestry. For example, in some embodiments, the non-genetic covariates include one or more of weight, height, behavioural characteristics, medical traits and other biomarkers, such as blood or urine based measurements.

In some embodiments, the method further comprises outputting the genetic contribution to the risk. This allows downstream application of the genetic contribution.

In some embodiments, the risk is a relative risk in relation to an individual having an average estimated genetic contribution, and the method further comprises: calculating the relative risk for the target individual for the target phenotype or target phenotype combination using the genetic contribution and a non-genetic contribution to the relative risk; and outputting the relative risk. The relative risk is a measure that can be more directly correlated to an individual's chance of developing a phenotype.

In some embodiments, calculating the relative risk comprises using a loss function to determine a value of the relative risk for the target individual from a distribution of the relative risk for the target individual. The loss function determines an appropriate selection of a single value as an expected value from the distribution. For example, in some embodiments the loss function is a mean-squared error function or an asymmetric exponential loss function.

In some embodiments, the method further comprises: calculating an absolute risk for the target individual for the target phenotype or target phenotype combination using the genetic contribution calculated using the method of any preceding claim; and outputting the absolute risk. An absolute risk is another useful measure for expressing an individual's chance of developing a particular phenotype.

According to a second aspect of the invention, there is provided an apparatus for analysing genetic data according to claim 15. The processor may be further configured to carry out analogous operations as described for the computer-implemented method above.

The invention may also be embodied in a computer program comprising instructions which cause the computer to carry out the method, or a computer-readable medium comprising instructions which, when executed by a computer, cause the computer to carry out the method.

Embodiments of the invention will be further described by way of example only with reference to the accompanying drawings, in which:

Fig. 1 shows the estimated odds ratio per unit of PRS according to a prior art method;
Fig. 2 is a flowchart of the method according to an embodiment of the invention;
Fig. 3 shows training data missing South Asian individuals projected into the ancestry space;
Fig. 4 shows effect size estimates for different ancestries under a linear, absolute position model of the genetic contribution using the training data of Fig. 3;
Fig. 5 shows maximum likelihood estimates for different terms in the genetic contribution under the same model and conditions as Fig. 4
Fig. 6 shows effect size estimates similar to Fig. 4 where the genetic contribution uses fewer dimensions of the ancestry space;
Fig. 7 shows the estimated odds ratio per unit of PRS for different ancestries under a linear, relative position model of the genetic contribution for the same training data as Figs. 3-6;
Fig. 8 shows effect size estimates for different ancestries under the same model as Fig. 7;
Fig. 9 shows effect size estimates for different ancestries under a hierarchical linear, absolute position model of the genetic contribution for the same training data as Figs. 3-8;
Fig. 10 shows effect size estimates for different ancestries under a non-linear, absolute position model of the genetic contribution for the same training data as Figs. 3-9;
Fig. 11 shows effect size estimates for different ancestries under a Gaussian process, relative position model of the genetic contribution for the same training data as Figs. 2-7.

## Detailed Description

*State of the Art*

As mentioned above, the mapping from weighted genetic variants in the form of a PRS to relative risk for an individual is not straightforward. The challenge is extrapolation: based on limited case-control or prospective datasets from particular populations, it is necessary to extrapolate the PRS effect size to individuals who either originate from different populations, or who do not exactly fit the features of the training set.

The simplest approach, is to assume that the effect size of the standardised polygenic risk score (PRS) is constant across all populations, or equivalently, that the phenotypic variance explained by the standardised PRS is independent of ancestry. This is demonstrably false, but may provide a reasonable starting point when limited data is available. This results in the following model:

$$Y_i \sim Bernoulli(\pi_i)$$
$$logit(\pi_i) = \beta_0 + \beta_{PRS} X_{PRS,i} \qquad (1)$$

where $Y_i$ is a random variable indicating if individual i has a disease (1) or not (0), $\pi_i$ is the probability of individual i being a case, $X_{PRS,i}$ is the PRS of individual i, $\beta_{PRS}$ is the effect size of the PRS, and $\beta_0$ is a constant factor. Other covariates could be included, but have been omitted here for clarity. Some existing approaches include principal components (PCs, discussed in more detail below) or a proportion of European ancestry as covariates in this model, but without interaction with the PRS. This therefore still results in a single PRS effect size (Amariuta, T., Ishigaki, K., Sugishita, H. et al. 2020 Methods, Eq. 2, Fritsche et al. 2021 Methods, Eq. 1 and Bitarello and Mathieson 2021).

A second and more reasonable approach, which represents the current state of the art, consists of assigning individuals to one of a collection of predetermined ancestries, and fitting the model of Eq. (1) in each ancestry. This results in the following model:

$$Y_i \sim Bernoulli(\pi_i)$$
$$logit(\pi_i) = \beta_{0,k} + \beta_{PRS_k} X_{PRS,i} \qquad (2)$$

where $\beta_{PRS_k}$ is the PRS effect size of population k, and the constant factor is also allowed to vary between populations as $\beta_{0,k}$.

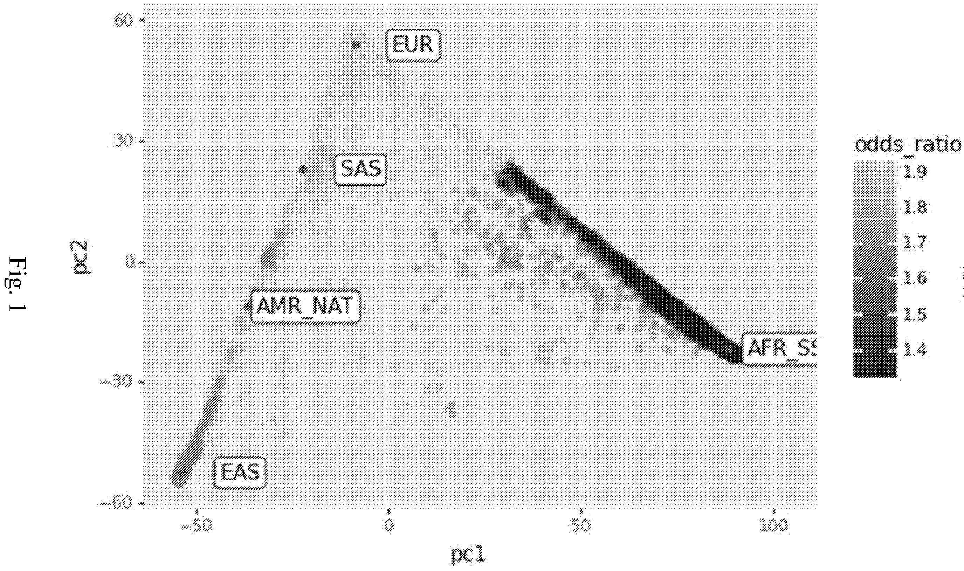

This approach is visualised in Fig. 1. Fig. 1 shows samples in a testing dataset projected onto the first two principal components (PCs) defined by the 1,000 Genomes dataset, and coloured by the estimated odds ratio (OR) associated to one standard deviation of PRS. In the case of the data shown in Fig. 1, the odds ratio per one standard deviation of PRS simply corresponds to e $\beta PRS k$ in model (2), because the PRS is standardised to have a standard distribution of 1.

Fig. 1 shows a projection of the individuals into an ancestry space using the PCs defined by the 1000 Genomes dataset. This type of projection into an ancestry space will be discussed further below in relation to the present invention, but is not typically done in existing approaches. The projection shown in Fig. 1 serves to demonstrate the existing approaches in a format that is easily compared to embodiments of the present invention discussed later.

As shown, each individual is assigned strictly (or hard-called) to one of five pre-defined ancestry groups, namely European (EUR), South Asian (SAS), Native American (AMR_NAT), East Asian (EAS), and African (AFR_SS). In other words, each individual is simply assigned to the ancestry group to which they are most similar by some measure. Odds ratios are estimated separately for each hard-called group, resulting in discontinuities along the continuous clines of ancestry between Europeans and East Asians and Europeans and Africans. Similar discontinuities exist along clines to South Asians and Native Americans along the axes defined by PCs 3 and 4, although this is not shown here. Red points denote cluster centres of samples in each of the five super-populations in 1,000 Genomes, defined by the mean of samples assigned to that group following removal of admixed samples and subsequent cluster assignment.

One previous study has determined distances in an ancestry space to demonstrate the attenuation of predictive performance, as predicted on theoretical grounds (Priv6 et al. 2021). However, the use of distances in an ancestry space has not been connected with the significance of PRS effect sizes, or used to correct risk scores based on ancestry. Some studies have attempted to quantify the extent to which PRS accuracy drops over genetic distance (Priv6 et al. 2021), but there has not been any development of methodologies to account for this phenomenon.

Incorrectly estimating the relative effect size creates uncalibrated datasets, that either over or under-estimate the role of genetics. This is potentially damaging as preventative or diagnostic measures may be applied to the wrong individuals. Estimating the correct effect size of the standardised PRS requires weighting the genetics of an individual appropriately, where the interpretation of the effect size on this scale is the change in log odds associated to a one standard deviation change in PRS. The present invention addresses these problems. To move away from grouping all individuals into discrete homogeneous groups we instead allow PRS effect size to vary with the location of the individual in ancestry space and build models that use this continuous definition of ancestry.

*Present Invention Introduction*

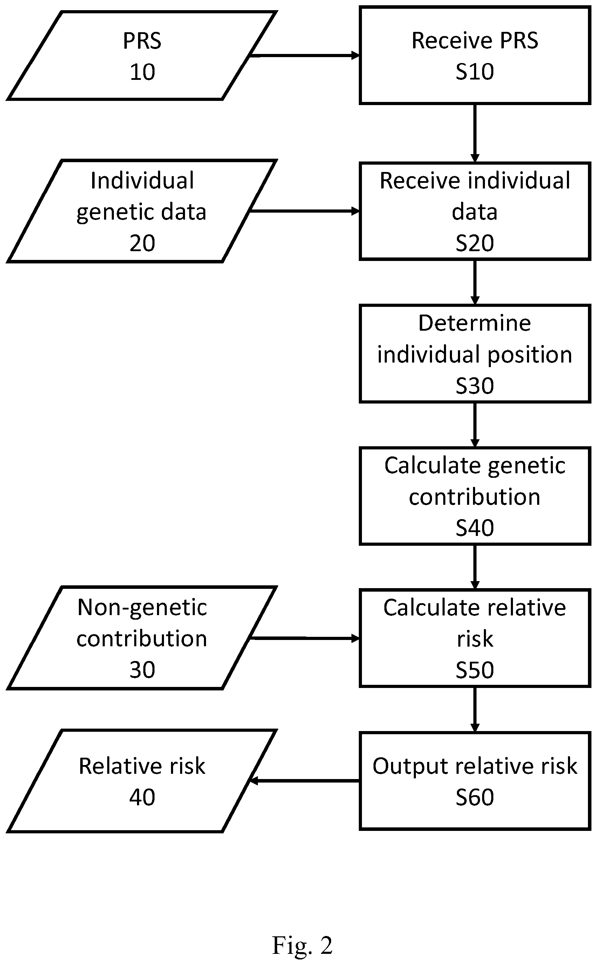

Fig. 2 shows an embodiment of a computer-implemented method of analysing genetic data. The method comprises receiving S10 a polygenic risk score (PRS) 10 for a target phenotype or target phenotype combination for a target individual. As mentioned above, a PRS is a quantitative summary of the effect of an individual's genetic variants on their risk for a particular phenotype or phenotype combination. The PRS 10 may be calculated using any suitable method. The calculation of the PRS 10 may be performed immediately prior to the present method, for example by the same computer system. Alternatively, the PRS 10 may be calculated elsewhere at a different time and transmitted to the system carrying out the present method. The PRS 10 may concern any phenotype. For example, the target phenotype could be a disease such as heart disease, cancer, diabetes, or any other disease of interest.

The method further comprises a step S20 of receiving individual genetic data 20 for the target individual. The individual genetic data 20 is informative about an ancestry of the target individual. For example, the individual genetic data 20 may comprise data about a plurality of genetic variants that are known to be indicative of an individual's ancestry. However, it is not necessary for the individual genetic data 20 to include information about genetic variants that are informative about the target phenotype or target phenotype combination. The information about the individual's genetics that relates directly to the target phenotype is already encoded in the PRS 10.

*Ancestry Space*

The method further comprises determining S30 an individual position in an ancestry space using the individual genetic data. The ancestry space may be defined using reference genetic data from a plurality of reference individuals having a plurality of different ancestries. For example, the reference genetic data may be derived from GWAS or publicly-available databases, such as the 1000 Genomes database mentioned in relation to Fig. 1. The reference genetic data is informative about the ancestries of the reference individuals. For example, the reference genetic data may comprise data for each reference individual about at least a plurality of genetic variants that are known to be indicative of an individual's ancestry.

The plurality of genetic variants about which the reference genetic data is informative may be the same or substantially overlap with (e.g. have at least 50% of the variants the same between the two pluralities) the plurality of genetic variants about which the individual genetic data is informative. By using the same genetic variants to define the ancestry space as are used for the individual, the accuracy of placement of the individual in the space can be improved.

Each reference individual may be assigned to one of a plurality of ancestries. For example, the reference individuals may be assigned to one of the previously-mentioned pre-defined ancestry groups, namely European (EUR), South Asian (SAS), Native American (AMR_NAT), East Asian (EAS), and African (AFR_SS). More or fewer pre-defined ancestry groups may be included depending on the reference genetic data being used. Labelling the reference individuals in this way helps to define the regions in the ancestry space associated with each ancestry group. Alternatively, only a subset of the reference individuals may be assigned to one of the plurality of ancestries. This may be preferable where the reference genetic data contains admixed individuals who do not closely match any of the pre-defined ancestry groups.

It is preferable to include data from as many reference individuals as possible in the reference genetic data, and from individuals having a wide range of ancestries. This will help to better define the ancestry space, and allow interpolation about the target individual to be made more confidently over a larger region of the ancestry space.

In some embodiments, a coordinate system of the ancestry space is determined by applying dimension reduction to the reference genetic data. It is known in human genetics to use dimension reduction statistical methodologies to map the highly multidimensional genetics space (with millions of variants) into a subspace having a smaller number of dimensions. This subspace is the ancestry space. The present invention is primarily concerned with using the position in the ancestry space to correct the effect size of the PRS. However, the choice of the ancestry space and how it is derived does have downstream effects on the models used for estimating the PRS effect size.

In some embodiments, the dimension reduction may comprise a discretisation of the ancestry space into a finite set of ancestries, and the individual position comprises a continuous or pseudo-continuous membership proportion for each ancestry of the finite set of ancestries. For example, the individual position may comprise an assignment to one or a weighted combination of a plurality of ancestries. By allowing the individual position to indicate a mixture of different discrete ancestries for the individual, a more accurate estimation of an individual's ancestry can be obtained relative to the 'hard-calling' approach of the state of the art illustrated in Fig. 1. This in turn allows for a more accurate estimate of the effect size of the PRS for the target individual.

The ancestry space is typically approximated through a dimensionality reduction of the reference genetic data to k dimensions. Typically a linear dimension reduction technique is used. For example, the dimension reduction may comprise principal component analysis (PCA), independent component analysis, non-negative matrix factorisation, or factor analysis. Non-linear dimension reduction techniques could also be used as long as it is possible to project new samples (i.e. the individual genetic data from the target individual) into the dimension-reduced subspace that is the ancestry space. For example, the ancestry space may be defined by performing principal components analysis (PCA) and taking the first k principal components (PCs). Typically the ancestry space may have 2, 3, or 4 dimensions. However, higher-dimensional ancestry spaces may also be used, for example having 5, 6, 7, 8, 9, 10, or more than 10 dimensions.

For the examples in the remainder of this application, the linear subspace spanned by the first four PCs is used.

In some embodiments, the coordinate system of the ancestry space is chosen to maximise the variance in the reference genetic data accounted for by the ancestry space. For example, where the dimension reduction comprises PCA, this may be achieved by selecting the PCs that account for the largest variance in the reference genetic data.

This choice means that the axes of the ancestry space best correspond to variations that reflect differences in ancestry. For example, where the reference genetic data comprises data on a plurality of genetic variants for each reference individual, the variance in the reference genetic data may be the variance among genetic variants that are known to be indicative of the ancestry of the individuals, or among variants that are seen to vary between ancestry groups within the data set.

In some embodiments, the ancestry space has a dimensionality lower than that of the individual genetic data, and determining the individual position comprises projecting the individual genetic data onto the ancestry space. Once the ancestry space has been determined, the individual position can be determined by projecting the individual genetic data on the ancestry space. The individual position may be represented in a variety of ways in the ancestry space. In some embodiments, the individual position may be represented by a combination of orderable variables, pseudo-continuous variables, or continuous variables. For example, the individual position may comprise an orderable variable, pseudo-continuous variable, or continuous variable for each axis or dimension of the ancestry space. The type of variable may be the same for all dimensions of the ancestry space, or may differ between the dimensions of the ancestry space.

It is often helpful if the PRS 10 is understood as the relative risk conditional on a position on the genetically defined ancestry space. A PRS is referred to as "centred" if the expectation (mean) of that PRS conditional on a position in the ancestry space is 0. A (centred) PRS is referred to as "standardised" if the variance of the PRS conditional on a position in the ancestry space, or population label is 1. It is preferred, although not essential, that a centred and standardised PRS is used, so that the PRS is reflective only of the relative risk for the individual and does not capture differences between ancestry groups.

A centred and standardised PRS can be viewed as a Gaussian with mean 0 and variance 1 at any point of the PC map (i.e. the ancestry space), due to the central limit theorem. Different but related techniques can be used to obtain that standardisation, but these are not the focus of this document.

*Calculating genetic contribution*

The method further comprises calculating S40 a genetic contribution to a risk for the target individual for the target phenotype or target phenotype combination using the polygenic risk score 10 and the individual position.

Once the individual position in the ancestry space has been determined, a relationship between the individual position and the effect size per unit PRS can be used to determine the genetic contribution to risk for the individual. The relationship between the individual position, PRS, and the genetic contribution to risk can be modelled in a variety of ways. In the present method, two broad approaches are considered. As mentioned above, the examples in this application define the ancestry space using the linear subspace spanned by the first four PCs defined from the 1000 Genomes project data.

The first broad approach is a multi-dimensional 'absolute position' approach. In such embodiments, the genetic contribution comprises a sum of sub-contributions for each axis of the ancestry space, each sub-contribution calculated using a coordinate of the individual position along the respective axis of the ancestry space. In other words, the coordinates of the individual position in each axis or dimension of the ancestry space all contribute independently in the dependence of the genetic contribution on the individual position. To obtain the dependence of the genetic contribution on the individual position and PRS, a model is fitted which includes the PRS, and each axis of the ancestry space (defined by the truncated dimensionality reduction of the reference genetic data), and their interactions, to obtain ancestry-specific estimates of the effect size contribution of PRS to risk.

In embodiments of such an 'absolute position' approach, the ancestry space may be non-isotropic, such that a dependency of each sub-contribution on the respective coordinate of the individual position differs between the sub-contributions. This allows maximum flexibility in fitting the dependence of the genetic contribution to account for different variations in the ancestry of the target individual, represented by changes in the individual position along different axes of the ancestry space.

The second broad approach is a single-dimensional 'relative position' approach. In such embodiment, calculating the genetic contribution comprises calculating a distance in the ancestry space between the individual position and a reference position in the ancestry space, and calculating the genetic contribution using the distance. In other words, the genetic contribution depends only on the absolute distance in the ancestry space between the individual position and a reference point. For example, the distance may be a Euclidean distance in the ancestry space. The genetic contribution may comprise a product of the polygenic risk score and the distance.

In this 'relative position' set of models, the absolute position of each sample in ancestry space is not directly incorporated into the dependence of the genetic contribution, but rather the relative distances between the target individual's individual position and some reference point are used, as some function of the axis of multi-dimensional ancestry space. In some embodiment, the reference position is a position in the ancestry space of an ancestry used to train coefficients used to calculate the polygenic risk score. In other words, the reference position corresponds to the ancestry of the training data used to create the PRS, i.e. to train (e.g. by a machine-learning algorithm) a set of coefficients that are used to obtain the PRS for the target individual based on their individual genetic data.

This 'relative position' approach is motivated by the observation that the variance explained by PRS decays monotonically with ancestral distance from the population used to fit the PRS. Thus, this relative distance and its interaction with PRS are included as covariates when fitting the model of the dependence of the genetic contribution. This allows us to estimate PRS effect sizes which vary continuously with ancestral distance from the population used to train the PRS.

In some embodiments, the ancestry space is defined using reference genetic data from a plurality of reference individuals having a plurality of different ancestries; and calculating the genetic contribution comprises scaling each axis of the ancestry space using a variance accounted for by the respective axis in the reference genetic data before calculating the distance. When using the relative position approach, scaling the axes of the ancestry space by the variance they explain ensures that coordinates that are more significant are more heavily weighted in the relative position.

In either the first (multi-dimensional) or the second (single-dimensional) approach, the genetic contribution may have a continuous or pseudo-continuous dependency on the individual position. Further, for each of the multi-dimensional and single-dimensional approaches, both linear and non-linear dependencies of the genetic contribution on PRS and individual position are considered. Specifically, logistic regression-based estimates are considered, which implicitly assume a linear dependence between individual position and the change in logarithm of the odds ratio, and non-linear based estimates of ancestry-specific PRS using Gaussian processes and general additive models (GAMs).

*Linear Models*

In some embodiments, calculating the genetic contribution comprises using a linear dependency on the individual position. As mentioned above, this can incorporate the individual position as either a multi-dimensional absolute position, or a single-dimensional relative position.

<u>Absolute position</u>

In some embodiments, the genetic contribution comprises a sum of sub-contributions for each axis of the ancestry space, each sub-contribution calculated using a coordinate of the individual position along the respective axis of the ancestry space. A straightforward method to incorporate an ancestry dependent effect size PRS estimate is to include the individual's PRS, the individual position (in terms of the principal components in this example), and their interactions as covariates in the following logistic model.

$$Y_i \sim Bernoulli(\pi_i)$$

$$logit(\pi_i) = \beta_0 + \beta_{PRS}X_{PRS,i} + \sum_{j=1}^{k} \left( \beta_{PC_j} \; X_{PC_j,i} + \beta_{PRS \times PC_j} \; X_{PRS,i}X_{PC_j,i} \right) \quad (3)$$

where, as above, $Y_i$ is a random variable indicating if individual i has a disease (1) or not (0), $\pi_i$ is the probability of individual i being a case, $X_{PRS,i}$ is the PRS of individual $i$, $\beta_{PRS}$ is the effect size of the PRS alone, and $\beta_0$ is a constant factor. In addition, $X_{PC_j,i}$ is the coordinate of the individual position for individual i for the dimension (axis) of ancestry space corresponding to PC $j$, $\beta_{PC_j}$ is the effect size for the position alone, and $\beta_{PRS \times PC_j}$ is the effect size for the combination of position and PRS. Each sub-contribution comprises a product of the polygenic risk score and the coordinate of the individual position along the respective axis of the ancestry space.

In this model, we assume some overall effect size in the absence of ancestral information, which is then modified by the individual position in ancestry space. Given that PRS is standardised to have mean 0 and variance 1, independent of ancestral location, we can then evaluate an ancestry specific PRS effect size $\beta_{PRS,i}$ for individual i, being the effect size for one standard deviation of PRS:

$$\beta_{PRS,i} := \beta_{PRS} + \sum_{j=1}^{k} \beta_{PRS \times PC_j} X_{PC_j,i}$$

This simplifies the equation for model (3) above to

$$logit(\pi_i) = \beta_0 + \beta_{PRS,i}X_{PRS,i} + \sum_{j=1}^{k} \left( \beta_{PC_j} \; X_{PC_j,i} \right)$$

Given the location of cluster centres in PC space, we can use this formula to compare to the state of the art categorical approach given by model (2) above. Using model (3), it is also simple to evaluate confidence intervals for the PRS effect size estimates under the present model using the Wald statistic.

$$L = \left[ 0, 1, 0_{1 \times k}, X_{PC_1,i}, \ldots, X_{PC_k,i} \right]$$

$$SE = \sqrt{LVL^\mathsf{T}}$$

where V is the covariance matrix. Then,

$$CI_{95\%} = \beta_{PRS,i} \pm 1.96 \times SE$$

Model (3) is simple, extremely fast, and provides a continuous or pseudo-continuous extension to the state of the art method of discrete assignment to ancestral categories. As the underlying model of the latent risk is linear, the interpretation of one unit of standardised PRS does not vary with location on the PRS scale.

In this and other embodiments, a dependency of the genetic contribution on the individual position and the polygenic risk score is determined using training data from a plurality of training individuals having a plurality of different ancestries. The training data comprises, for each of the training individuals, genetic data, and whether the training individual has the target phenotype or phenotype combination.

It is worth noting that the training data used to train the model of the dependence of the genetic contribution on the PRS and the individual position may differ from the reference genetic data used to define the ancestry space. For example, the reference genetic data may only contain information on genetic variants relevant to the determination of genetic ancestry, and may not contain sufficient data relevant to the target phenotype to allow it to also be used to train the model. In some embodiments, the reference genetic data and the training data may be the same, but this is not always true.

Extrapolation to regions of ancestry space not represented in the training data can lead to spurious results, so care should be taken to interpolate and not extrapolate. If a super-population (substantially corresponding to one of the pre-defined ancestry groups, for example) is not present in the training data but is present in the reference genetic data, which is assumed to capture worldwide genetic diversity, then a single PC will distinguish that super-population from the others. Moreover, the remaining individuals from the other super-populations will likely not differ to a great extent along the axis of the ancestry space defined by that PC. This results in instability in the effect size estimate of that single PC, and vulnerability to extreme estimates.

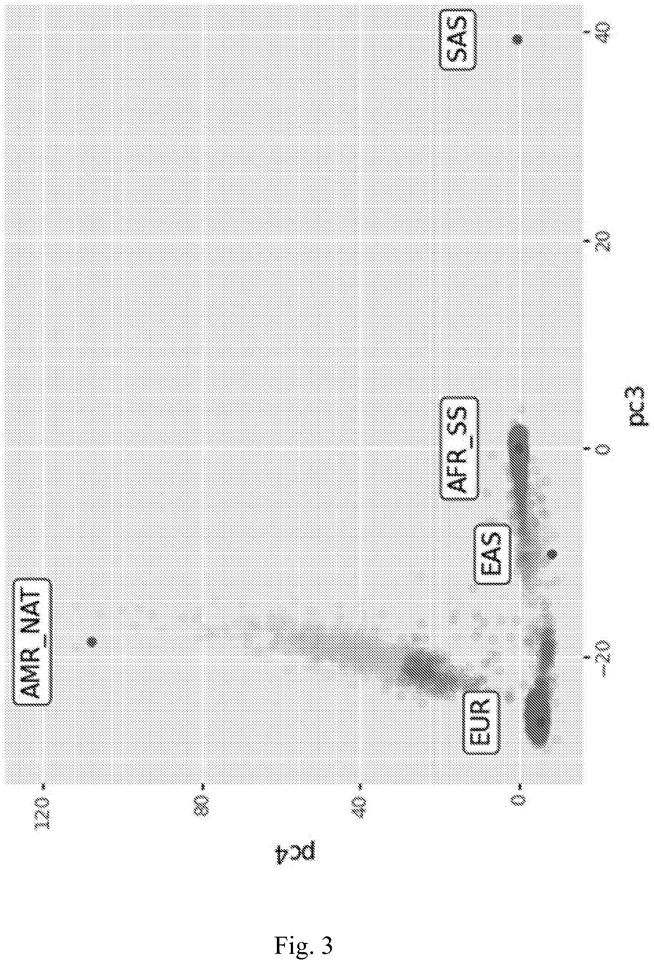

For example, Fig. 3 shows an example of training data projected into the PC3/PC4 plane of the ancestry space (defined by the first four PCs from 1000 Genomes), where the training data lacks individuals of SAS ancestry. Solid red points and labels in Fig. 3 show the centroids of each super-population from the 1000 Genomes data. As can be seen, the training lacks any samples around the SAS centroid.

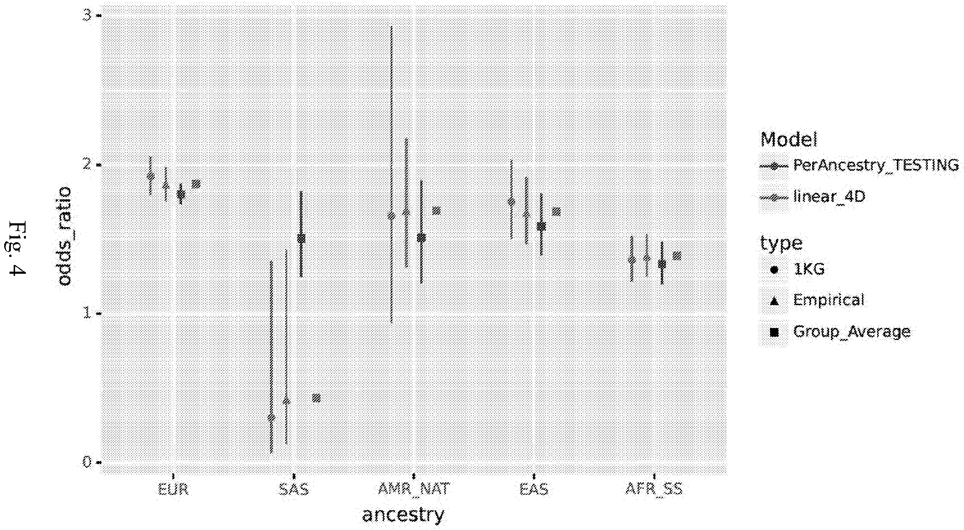

Fig. 4 shows effect size estimates (the estimated effect size per unit PRS) for an individual at the average location of each of the five major super-populations in 1000 Genomes under model (3), where the model is trained using the training data of Fig. 3. Effect size estimates are reported for each super-population using the first four PCs in a logistic regression (blue), and compared to the discrete estimate within that population in testing (red). The discrete estimate is the estimate calculated using only the testing data from that super-population, rather than using the complete testing data from all super-populations with appropriate corrections for their position in the ancestry space. Blue circles ("1KG") refers to the odds ratio at the median PC location of the super-population in 1000 Genomes, blue triangles ("Empirical") refer to the odds ratio at the mean PC location of the testing set individuals within a given super-population . The blue square point estimate ("Group Average") represents the average effect size estimate across testing set individuals within each super-population label. Due to the lack of SAS individuals in the training data, the estimated effect size for an individual at the SAS position is below the reasonably expected minimum bound of 1.0.

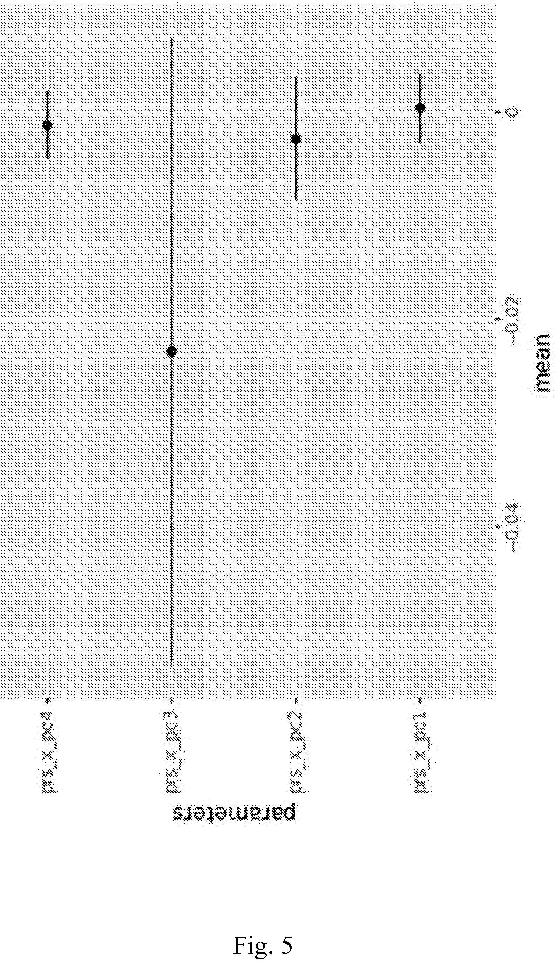

Fig. 5 shows the maximum likelihood-based estimates (MLE) of the interaction coefficients ($\beta_{PRS \times PC_j}$) in model (3)) and their associated 95% confidence intervals. For each interaction coefficient, the MLE is shown as a black dot, and the associated standard errors as whiskers. As expected, given the paucity of training data along PC3 (due to the lack of the SAS super-population), standard errors for the interaction coefficient in the logistic fit of model (3) are large. This leads to the unreasonably low effect size estimate for a SAS individual in Fig. 4, due to the large negative point estimate of PC3 (for which South Asian individuals have the largest weighting) in Fig. 5.

To guard against this type of unstable estimation, a number of alterations can be considered. One approach is to determine the PC which separates the super-population that is absent from the training data from the other super-populations in the ancestry space and remove that PC as a covariate (and its corresponding interaction coefficient $\beta_{PRS \times PC_j}$ with the PRS) in model (3). However, this approach is biased to overestimate the effect size because the model "thinks" new, target individuals in the missing super-population are much closer in ancestry space to the individuals in the training data than they actually are.

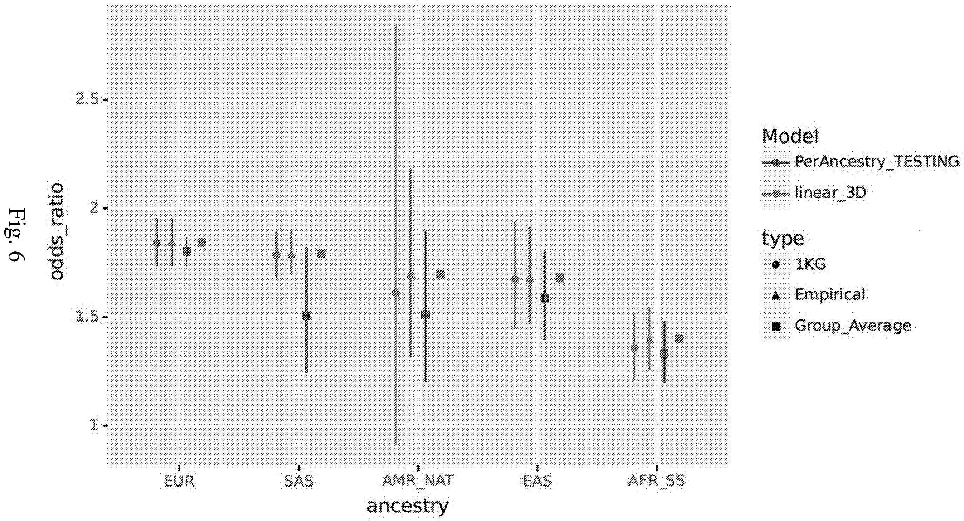

This approach to the correction is shown in Fig. 6, continuing with the same data as used in the previous examples of Figs. 3-5. Fig. 6 shows the effect size estimates for an individual at the average location of each super-population under model (3) in an ancestry space defined by only three PCs. Effect size estimates are reported for each of the five major super-populations in 1000 Genomes using the three PCs in a logistic regression according to model (3) after removing the PC which distinguishes South Asians from the remaining super-populations (blue) in a model with the first four PCs, and compared to the discrete estimate within that population in testing (red). Blue circles ("1KG") refers to the odds ratio at the median PC location of the super-population in 1000 Genomes and blue triangles ("Empirical") refer to the odds ratio at the mean PC location of the testing set individuals within a given super-population. The blue square point estimate ("Group Average") represents the average effect size estimate across individuals within each super-population label. As can be seen, the odds ratios for SAS are higher than the discrete estimate within the super-populations in testing.

An alternative approach is to introduce regularisation terms into the model of the dependence of the genetic contribution to guard against overfitting, for example using LASSO, elastic net, or ridge regression.

Relative Position

In some embodiment, calculating the genetic contribution comprises calculating a distance in the ancestry space between the individual position and a reference position in the ancestry space, and calculating the genetic contribution using the distance. In this case, the effect size can be modelled as varying continuously with ancestry using the following model:

$$Y_i \sim Bernoulli(\pi_i)$$
$$logit(\pi_i) = \beta_0 + \beta_{PRS} X_{PRS,i} + \beta_{EUR} X_{EUR,i} + \beta_{PRS \times EUR} X_{PRS,i} X_{EUR,i} \tag{4}$$

where $X_{EUR,i}$ is the genetic distance from individual i to the median position of the EUR super-population in the ancestry space. In what follows, this distance is defined as the Euclidean distance following a scaling of the first four PCs by the variance they explain in the reference genetic data. In other words, the ancestry space is defined using reference genetic data from a plurality of reference individuals having a plurality of different ancestries; and calculating the genetic contribution comprises scaling each axis of the ancestry space using a variance accounted for by the respective axis in the reference genetic data before calculating the distance. However, the method is not limited to these choices - more or fewer PCs could be included, and a non-Euclidean distance measure could be used.

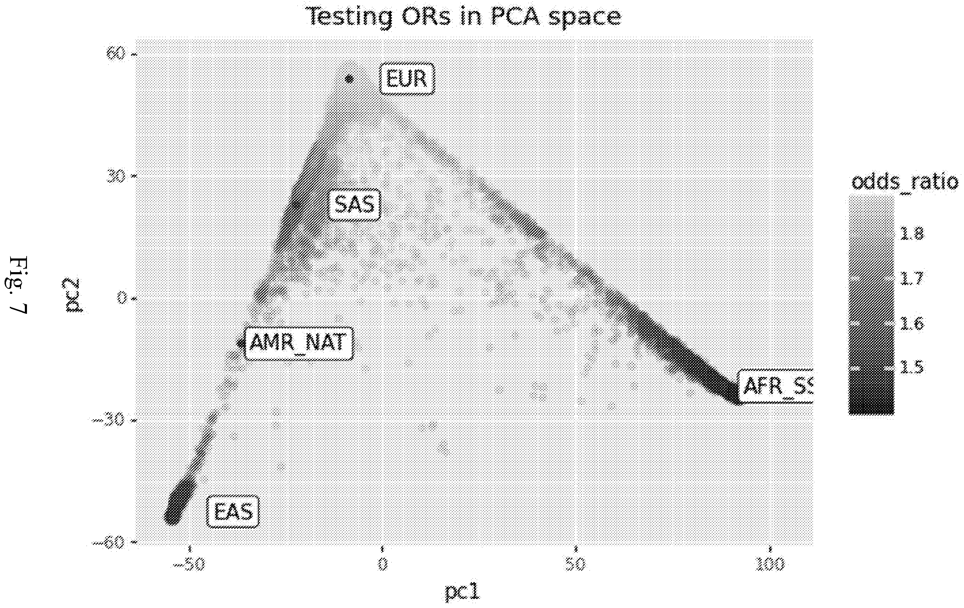

Fig. 7 shows the same example training data as used in Figs. 3 to 6. This time, the data is projected into a plane in the ancestry space defined by PC1 and PC2. Each point is a training individual in the training data, coloured by their estimated PRS effect size ("odds ratio") according to model (4). In the linear models of the present method, there are no discontinuities unlike the state of the art approach shown in Fig. 1.

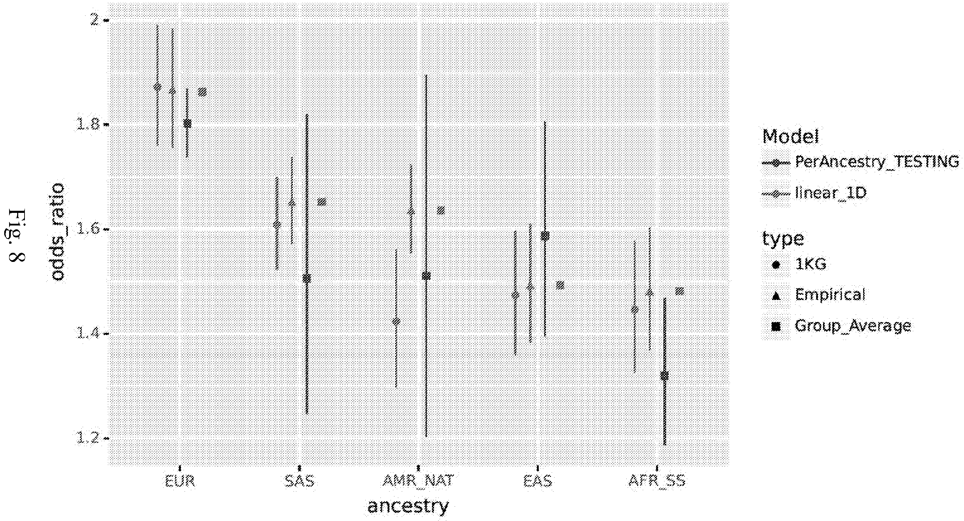

Model (4) results in close agreement with the testing set SAS effect size estimates, as shown in Fig. 8. In Fig. 8, effect size estimates are reported for each of the five major super-populations in 1,000 Genomes using the three PCs in a logistic regression according to model (4), and compared to the discrete estimate within that population in testing (red). Blue circles ("1KG") refers to the odds ratio at the median PC location of the super-population in 1000 Genomes and blue triangles ("Empirical") refer to the odds ratio at the mean PC location of the testing set individuals within a given super-population. The blue square point estimate ("Group Average") represents the average effect size estimate across individuals within each super-population label.

However, the testing estimates could be different due to cohort/environmental effects or large uncertainty due to small sample sizes (as in the AMR_NAT case). Note that the EAS effect size estimate is slightly lower and AFR_SS slightly higher compared to the previous approaches, which may reflect the less flexible nature of this model.

Model (4) (and other similar, 'relative position' models) assumes that the PRS effect size changes in the same way when the individual position changes, regardless of the axis in the ancestry space along which the individual position moves. This is a reasonable assumption, because it has previously been observed that the prediction discrimination/accuracy drops approximately linearly with distance in ancestry space. In addition, similar effect sizes are observed when binning individuals by distance.

Model (4) has the same advantages as the multi-dimensional linear model. However it also has the additional benefit that, because the ancestry space is effectively reduced to a single dimension for the purpose of the model of the genetic contribution, it is much less likely that a new individual will have a position in the extrapolation regime (i.e. outside the region covered by training data). Rather, the model will be interpolating between training individuals in the individual training data, resulting in more stable estimates of the model parameters (i.e. the interaction coefficients). This effectively regularises the interaction coefficient.

A limitation of model (4) is that it makes an additional assumption compared to the multi-dimensional model (exemplified by model (3)). This means the model is less flexible and has fewer degrees of freedom. It is possible that the assumption that PRS effect size changes in the same way in every direction in ancestry space may be inaccurate. In this case, the relative position model would be underfitting the training data, resulting in biased estimates. In addition, the relative position model also requires specification of a reference point. A similar extrapolation issue exists for this model if African samples are not present in the training data used to fit the model, though it is likely more robust than the multi-dimensional model to such an absence of training data.

*Hierarchical model*

Models (3) and (4) can be viewed as extremes of a spectrum. At one end of the spectrum (model (3)), each PC interaction coefficient is estimated without sharing information between the axes of variation in the ancestry space. In this case, the sub-contributions for each axis of the ancestry space are independent of one another. At the other end of the spectrum (model (4)), the axes of the ancestry space are collapsed into a single distance. In effect, this distance completely shares information across the PCs.

Between these two extremes is a 'hierarchical' model, which shares information across the different interaction terms and PCs whilst retaining the flexibility to allow different coefficients for each one. This effectively provides a number of degrees of freedom intermediate between one (as for 'relative distance' models) and the number of PCs (for 'absolute distance' models). In such embodiments, at least two of the sub-contributions have dependencies on the respective coordinates of the individual position that are related by a shared prior. These models use a shared prior across the interaction coefficients. This enables sharing of information and represents the prior belief that the PRS effect size should change in a similar way along the axis in ancestry space corresponding to each PC, while allowing that the change may not be identical along each axis. In some embodiments, the model can learn from the training data how similar the changes along each axis are to one another.

Relating the dependencies of the sub-contributions by the shared prior has some similarity to a standard hierarchical linear model (also known as a multi-level model) which shares information across groups of "individuals". However, in the present case, information is shared across the interaction coefficients themselves (which represent the dependencies of the sub-contributions on the respective coordinates) because the model has moved into the continuous ancestry space.

The present hierarchical model also differs from a standard hierarchical model in that, because each interaction coefficient has the same number of data points, the model relies on differing uncertainty of the interaction coefficients to inform how much each interaction coefficient should be regularised.

For a PC distinguishing a super-population which is present in the reference genetic data used to define the ancestry space, but which is missing in the training data, the estimate will be more uncertain due to the smaller range over which the gradient is learnt. As a result, these estimates will be regularised more strongly.

This model can be expressed as follows:

$$Y_i \sim Bernoulli(\pi_i)$$

$$logit(\pi_i) = \beta_0 + \beta_{PRS}X_{PRS,i} + \sum_{j=1}^{k} \left( \beta_{PC_j} X_{PC_j,i} + \beta_{PRS \times PC_j} X_{PRS,i}X_{PC_j,i} \right)$$

$$\beta_{PRS \times PC_j} = \sigma_{PRS \times PC}\beta^*_{PRS \times PC_j} + \mu_{PRS \times PC}$$

$$\beta^*_{PRS \times PC_j} \sim N(0,1)$$

$$\mu_{PRS \times PC} \sim N(a,b)$$

$$\sigma_{PRS \times PC} \sim halfCauchy(c,d)$$

$$(5)$$

which implies:

$$\beta_{PRS \times PC_j} \sim N(\mu_{PRS \times PC}, \sigma^2_{PRS \times PC})$$

where *a, b, c,* and d are hyperparameters.

As for model (3), each sub-contribution comprises a product of the polygenic risk score and the coordinate of the individual position along the respective axis of the ancestry space. However, now the sub-contributions are related to one another by the shared prior.

As for the other models, the data and examples given in the figures only use the first four principal components, but in general any number could be included. Increasing this number to the total number of signal PCs in the reference genetic data could aid in the learning of the variance parameter $\sigma_{PRS \times PC}$.

As the signs of the principal components are arbitrary, these need to be harmonised relative to the training data, otherwise the learnt $\sigma_{PRS \times PC}$ will be artificially inflated, for example due to one coefficient being -0.1 and the other +0.1. This can result in a loss of information sharing, and failure to sufficiently regularise. The harmonisation may be performed by changing the signs of the principal components (e.g. by multiplying by minus one) such that the signs of all the learnt interaction coefficients are the same. Since the signs of the principal components are arbitrary, the sign chosen (positive or negative) does not matter as long as all are consistent.

The $\beta_{PRS \times PC_j}$ are sampled from a distribution, whose parameters are themselves learnt using information from both the data itself as well as the priors on these parameters themselves (which have hyperparameters set by the user). This is how the shared prior affects the definition of the dependencies of the sub-contributions. In effect, the shared prior is specified such that the dependencies of the at least two sub-contributions are sampled from the same distribution. One set of samples from the posterior distribution is taken for each principal component, not for each target individual or training individual. The result is a posterior distribution for each $\beta_{PRS \times PC_j}$.

The shared prior is determined using training data from a plurality of training individuals and one or more predetermined hyperparameters. The strength of the prior belief that the interaction coefficients should be similar can be varied by varying the hyperparameters. In the specific implementation exemplified above, the hyperparameter d can be varied, with smaller values of d corresponding to a stronger belief that they are similar.

From the posterior distribution for the interaction coefficients $f\beta_{PRS \times PC_j}$, a distribution of the odds ratio can be calculated for each training individual (or the target individual). Each posterior sample for an individual i is the sum of the product of the predictors (with $X_{PRS,i} = 1$ to obtain the effect size per unit PRS) and the posterior estimates of the coefficients, i.e. $\beta_{PRS} X_{PRS,i} + \sum_{j=1}^{k} (\beta_{PC_j} X_{PC_j,i} + \beta_{PRS \times PC_j} X_{PRS,i} X_{PC_j,i})$. So the full distributions are given by $A \cdot B$ where $A$ is an $N \times M$ matrix for $N$ individuals and $M$ predictors $[X_{PRS,i}, X_{PRS,i}X_{PC_1,i}, X_{PRS,i}X_{PC_2,i}, ...]$ (as above, with $X_{PRS,i}$ set to 1 to obtain a "per 1 PRS unit" odds ratio) and $B$ is an $M \times P$ matrix, where $P$ is the number of posterior samples.

A point estimate for each individual can then be derived given some loss function, commonly mean squared error, but could be another e.g. asymmetric exponential loss if over-estimation is considered worse than under-estimation or vice versa.

The model is shown in an "un-centred" version which helps in fitting the parameters of the model, especially the grand $\sigma_{PRS \times PC}$. However, the model could be formulated in a "centered" version which is equivalent and shown as an implied model. The non-interaction PC coefficients $\beta_{PC_j}$ do not directly affect the effect size, but rather the absolute baseline probability in an area of ancestry space. This means that the argument for these being similar is weaker than for the interaction coefficients $\beta_{PRS \times PC_j}$. However, the non-interaction coefficients could also be related by a hierarchical prior. Where no prior is stated in the models above, a uniform prior or a weakly informative prior is assumed.

The parameters in this Bayesian model (5) are fitted using Hamiltonian Monte Carlo to obtain the example results shown in the figures. However, other approximations could also be used, for example variational inference or integrated nested Laplace approximation. When fitted using Hamiltonian Monte Carlo, the model is slower than the other linear approaches, though faster than the variational Gaussian process discussed further below.

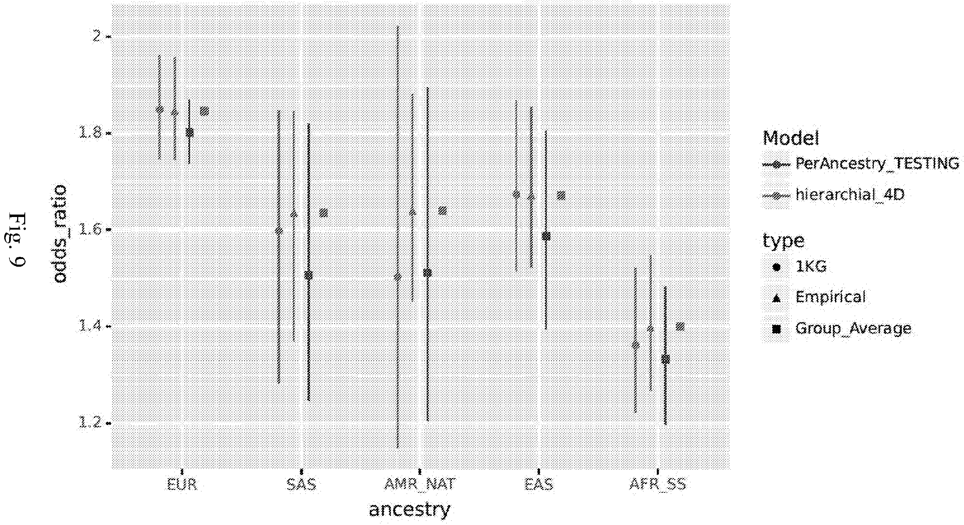

Fig. 9 shows effect size estimates for an individual at the average location of each super-population under model (5). Effect size estimates are reported for each of the five major super-populations in 1,000 Genomes using model (5) (blue), and compared to the discrete estimate within that population in testing (red). Blue circles ("1KG") refers to the odds ratio at the median PC location of the super-population in 1000 Genomes and blue triangles ("Empirical") refer to the odds ratio at the mean PC location of the testing set individuals within a given super-population. The blue square point estimate ("Group Average") represents the average effect size estimate across individuals within each super-population label.

Model (5) regularises enough to generate a SAS effect size very close to the discrete estimate within the testing set, as in the single dimensional model above. However, model (5) is also flexible enough to have an effect size estimate for EAS/AFR_SS similar to the discrete estimate within the testing set, as in the other multidimensional models. Model (5) strikes a balance between the single-dimensional (relative position) models and multi-dimensional (absolute position) models by learning how similar the interaction coefficients are from the training data, rather than hard coding an identical effect or allowing the effects to be "free". If the training data suggests that the dependencies of the sub-contributions along different axes of the ancestry space are really different, then a large standard deviation will be learnt and the dependencies will not be regularised as strongly.

**Non-Linear Models**

All of the models discussed so far have been linear models, where the dependencies of the genetic contribution (or its sub-contributions) on the individual position in the ancestry space have been linear. This means that a change of a certain size and direction in the individual position has the same effect on the genetic contribution regardless of where in the ancestry space it occurs. However, we can also consider non-linear models. In such embodiments, calculating the genetic contribution comprises using a non-linear dependency on the individual position.

*Generalised Additive Models (GAMs)*

A first example of a non-linear model is a generalised additive model (GAM). In the case of a GAM, instead of having a simple linear dependence between each coefficient and covariate (PRS or individual position), we can model this dependence as any function of each covariate:

$$Y_i \sim Bernoulli(p_i|X)$$
$$logit(p_i|X) = \beta_0 + f_1(X_1) + f_2(X_2) + \cdots + f_M(X_N) \tag{6}$$

The non-linear dependency may comprise a regularised function. This ensures that the dependency is smooth and does not contain sudden discontinuities or other features that are unlikely to be biologically reasonable. A common functional form to choose are penalised B splines (aka P-splines), which allow modelling non-linear forms. In such an embodiment, the non-linear dependency comprises a penalised B-spline. In a penalised B-spline, a penalty ("lambda") is placed on the curvature of the function, with the function becoming completely linear with larger values. This lambda value may be learnt using cross validation over a grid of values. One way to choose the preferred lambda value is to choose the value which minimises the unbiased risk estimator.

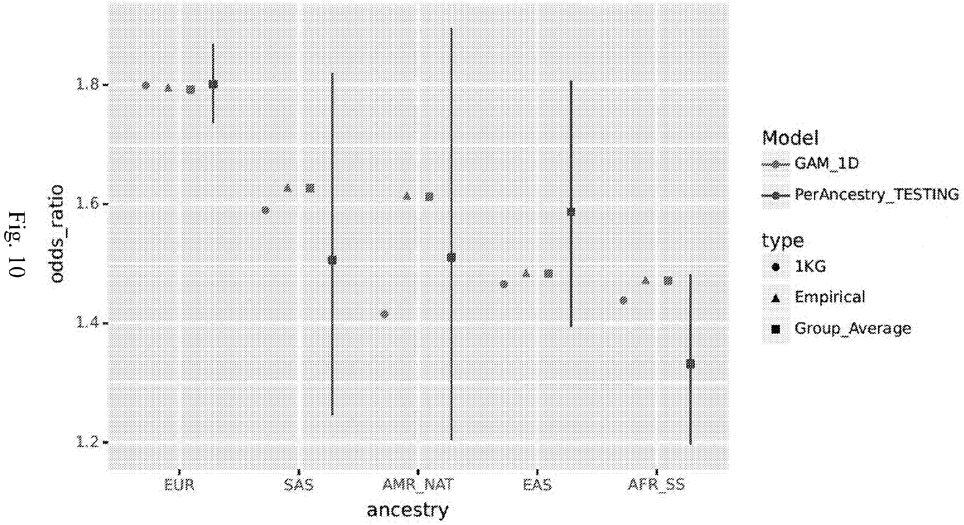

As an example of this type of model, Fig. 10 shows effect size estimates for an individual at the average location of each super-population under model (6). A single-dimensional, relative position model is used with a single distance from EUR and its interaction with PRS. Effect size estimates are reported for each of the five major super-populations in 1,000 Genomes using model (6) ( blue ) , and compared to the discrete estimate within that population in testing ( red). Blue circles ("1KG") refers to the odds ratio at the median PC location of the super-population in 1,000 Genomes, blue triangles ("Empirical") refer to the odds ratio at the mean PC location of the testing set individuals within a given super-population. Blue square points ("Group Average") denote the average effect size estimate across testing set individuals with each super-population label.

The results using model (6) are very similar to the single dimensional model results shown in Fig. 8. Model (6) is faster than the variational Gaussian process strategy discussed below, but still flexibly allows for non-linear dependencies. However, grid searching across different lambda values to fit the penalised B-spline example in Fig. 10 is slow, which limits the grid spacing that can be used to test different lambda values.

*Gaussian processes*

An alternative way to fit a non-linear dependence is to use Gaussian processes. In such embodiments, the non-linear dependency is determined using a Gaussian process as a prior distribution for calculating the genetic contribution using Bayesian inference.

In the case of Gaussian process regression, the following setup is assumed. The training data comprises a collection of observations $[y_1, y_2, ..., y_n]$ from some unknown function $f: X \to R$ at a collection of locations $[x_1, x_2, ... , x_n]$ with noise added:

$$y_i = f(x_i) + \varepsilon_i \text{ for } i = 1, 2, ..., n.$$

where $\varepsilon_i$ is a noise term.

From this training data, an estimate of the unknown function f for new data that arrives: $y^* = f(x^*) + \varepsilon^*$ is to be

determined. To do this, it is assumed that the data is sampled from a stochastic process, known as a Gaussian process (GP). Samples taken from a GP have functions $\mu(.)$, and $k(.,.)$ such that the expectation function $E[f(x)] = \mu(x)$ and the covariance function $Cov[f(x), f(x')] = k(x, x')$. The shorthand for this sampling procedure is $GP(\mu(.), k(.,.))$.

Each finite subset of a GP (in particular the subset of the data that is used for training) is sampled from a multivariate Gaussian distribution. How to interpolate between the points of the training data is determined by the choice of the functions $\mu(.)$, and $k(.,.)$. In the present situation, there is the added complexity that $Y$ is not continuous, but a discrete case/control label. It is assumed that $Y$ is Bernoulli distributed, and the underlying latent distribution governing the risk of being a case is sampled from a GP.

$$Y_i \sim Bernoulli(\pi_i)$$
$$logit(\pi_i) = GP(\mu(.), k(\cdot, \cdot)) \qquad (7)$$

There is also the additional issue that standard methods to evaluate the predictive means and variances have computational complexity $O(n^3)$, where $n$ is the number of data points. This is not an issue when n is on the order of thousands, but renders standard approaches intractable when sample sizes approach hundreds of thousands, as can be the case for genetic data.

To estimate the effect size per PRS for an individual, we use the posterior distribution. A posterior distribution for the Bayesian inference is determined using the Gaussian process and training data from a plurality of training individuals having a plurality of different ancestries.

An issue with the introduction of a link function (the Bernoulli function) relating the latent space to case-control status Y is that the posterior distribution is no longer Gaussian. To maintain tractability, in some embodiments, the non-Gaussian posterior is approximated with a Gaussian. Therefore, determining the posterior distribution comprises approximating the posterior distribution as a Gaussian distribution. There are a series of methods that can be used to do this approximation. For example, a Laplace approximation, Expectation propagation, and Kullback-Leibler (KL) divergence minimisation. In the present embodiment, the latter approach is used, by approximating the exact posterior distribution $p$ by a Gaussian $q$, and minimising $KL(q\|p)$. This minimisation problem can be solved using Newton's method and is also $O(n^3)$. Full details of the variational approximation are described in detail in (Nickisch and Rasmussen 2008).

When using Gaussian processes, it is common to assume a zero mean function $\mu(.) = 0$, such that the Gaussian process is given by $GP(0, k(.,.))$. This choice simplifies fitting, and the mean can always be added back afterwards, since the shape of the Gaussian process is entirely determined by its covariance function. In such embodiment, the Gaussian process has a zero mean function. However, in some cases, it may be desirable to include a non-zero mean function, either before fitting or added back after fitting. For example, a non-zero mean function can be used to control the OR (effect size) per unit of PRS in regions of the ancestry space away from the training data, effectively as a prior on the PRS contribution in the absence of genetic data at that ancestral location. For example, the effect size seen in African ancestry individuals could be set as the minimum effect size across the ancestry space, on the basis that they represent the "worst-case scenario" for ancestry attenuation. Therefore, in some embodiments, the Gaussian process has a mean vector corresponding to a prior estimate of the genetic contribution to the risk for the target individual. This approach can make fitting more challenging due to the nonlinearity of the GP.

Most important to the behaviour of the Gaussian process is the choice of the kernel function $k(x, x')$. The kernel function describes a functional form for the expected covariance between any pair of locations $x$ and $x'$.

Optionally, the kernel function of the Gaussian process is a stationary function. A stationary function depends on the distance between two points, and not their absolute positions. This means that the behaviour of the Gaussian process is similar throughout ancestry space. The kernel function may additionally be isotropic, where it depends only on the magnitude of the distance between two points. Optionally, the kernel function of the Gaussian process decays to zero with decreasing similarity between samples. Choosing a decaying kernel function ensures that the value of the function decays back to the mean in regions of the ancestry space far from any training data. This can help to control and reduce unexpected behaviour of the function when a target individual has an individual position that is not in close proximity to training data in the ancestry space. For example, the kernel function may be a radial basis function or a rational quadratic covariance function. In the example embodiments below, a radial basis function (RBF) is used as the kernel:

Optionally, the kernel function of the Gaussian process depends on one or more hyperparameters. For example, the hyperparameters may comprise a hyperparameter associated with each of the polygenic risk score, the individual position, and an interaction between the polygenic risk score and the position. In the examples below, one variance hyperparameter $\theta$, and three length scale hyperparameters are used: $l_{PRS}$, $l_{PC}$, $l_{PRS \times PC}$ for the PRS, PCs, and PRS $\times$ PCs interactions, respectively.

The hyperparameters may comprise hyperparameters associated with a coordinate of the individual position along each axis of the ancestry space, i.e. corresponding to each of the PCs. The hyperparameters may comprise hyperparameters associated with an interaction between the PRS and each coordinate of the individual position. However, in this example, a single length scale hyperparameter is used for the individual position (the PCs) and a single length scale hyperparameter for the interaction between the PRS and the individual position (PRS × PCs). This is achieved by scaling the relative length scales of the PCs by the variance they explain in the reference genetic data. Therefore, the ancestry space is defined using reference genetic data from a plurality of reference individuals having a plurality of different ancestries (as discussed previously), and calculating the genetic contribution comprises scaling each axis of the ancestry space using a variance accounted for by the respective axis in the reference genetic data before calculating the distance. This scaling and use of a reduced number of hyperparameters increases speed, guards against overfitting, and enforces a robust notion of "distance" in ancestry space.

As for the other models discussed above, the Gaussian processes can be used with either an absolute position, multi-dimensional approach, or a relative position, single dimensional approach.

Absolute position

In the absolute position models, the genetic contribution comprises a sum of sub-contributions for each axis of the ancestry space, each sub-contribution calculated using a coordinate of the individual position along the respective axis of the ancestry space. To apply the Gaussian process framework to the prediction problem in this case, the sample corresponding to each of the training individuals is defined by a vector:

$$x_i = \left[ x_{i,PRS}, x_{i,PC}, x_{i,PC \times PRS} \right]$$

where $x_{i,PRS}$, $x_{i,PC}$, and $x_{i,PRS \times PC}$ are the standardised PRS, the vector defining the position in the ancestry space location, and the corresponding interaction for individual i, respectively. The kernel function using a radial basis function is then given by:

$$k(x_i, x_j) = \theta \, exp \left( -\frac{1}{2} \left[ \frac{|x_{i,PRS} - x_{j,PRS}|^2}{l_{PRS}^2} + \frac{||x_{i,PC} - x_{j,PC}||^2}{l_{PC}^2} + \frac{||x_{i,PRS \times PC} - x_{j,PRS \times PC}||^2}{l_{PRS \times PC}^2} \right] \right).$$

Appealing to Gaussian process categorisation allows non-linear effects of PCs to be naturally incorporated into the prediction of individual case status. Furthermore, depending on the functional form of the kernel, the Gaussian process can enforce that the effect size of the PRS is 0 away from the training data. This is a desirable property: in the absence of information (as stipulated by the kernel), it is expected that the estimate will be the prevalence of disease in the training data.

The benefit of employing Gaussian process categorisation is also a drawback. In this framework, changes in PRS can non-linearly affect case probability. Thus, the impact of the PRS on risk varies as a function of its value (unless a linear kernel is enforced for terms including the PRS). Fitting a GP can also be slow. The speed can be improved by using sparse variational Gaussian processes, or by performing an approximation via a dimensionality reduction. The former approach can be difficult to robustly optimise. However, recent papers have suggested approaches to increase robustness in Gaussian process regression, but not yet for categorisation. The radial basis function kernel is an appropriate choice, because the trained length scale hyperparameters result in estimates which rapidly return to a baseline level in the absence of data.

Relative Position

In the relative position approach, calculating the genetic contribution comprises calculating a distance in the ancestry space between the individual position and a reference position in the ancestry space, and calculating the genetic contribution using the distance. For this single-dimensional implementation of the GP framework, a different set of predictors is used. Instead of the predictors from model (4), i.e.

$$Y_i \sim Bernoulli(\pi_i)$$
$$logit(\pi_i) = \beta_0 + \beta_{PRS} X_{PRS,i} + \beta_{EUR} X_{EUR,i} + \beta_{PRS \times EUR} X_{PRS,i} X_{EUR,i} \tag{4}$$

a Gaussian process is used according to model (7) having a zero mean function and the following kernel function:

$$k(x_i, x_j) = \theta\, exp\left(-\frac{1}{2}\left[\frac{|x_{i,PRS} - x_{j,PRS}|^2}{l_{PRS}^2} + \frac{|x_{i,EUR} - x_{j,EUR}|^2}{l_{EUR}^2}\right.\right.$$
$$\left.\left.+ \frac{|x_{i,PRS \times EUR} - x_{j,PRS \times EUR}|^2}{l_{PRS \times EUR}^2}\right]\right). \qquad (8)$$

As in the multi-dimensional embodiment above, four hyperparameters are used in the kernel function. In this embodiment, these are denoted $\theta$, $l_{PRS}$, $l_{EUR}$ and $l_{PRS \times EUR}$. These hyperparameters govern the overall scaling and length scales over which information about the PRS, the distance from the reference point (in this example, the Euclidean distance from the European cluster centre determined as the position of the median European in the reference genetic data), and interaction between the PRS and the distance, respectively, are shared between pairs of points.

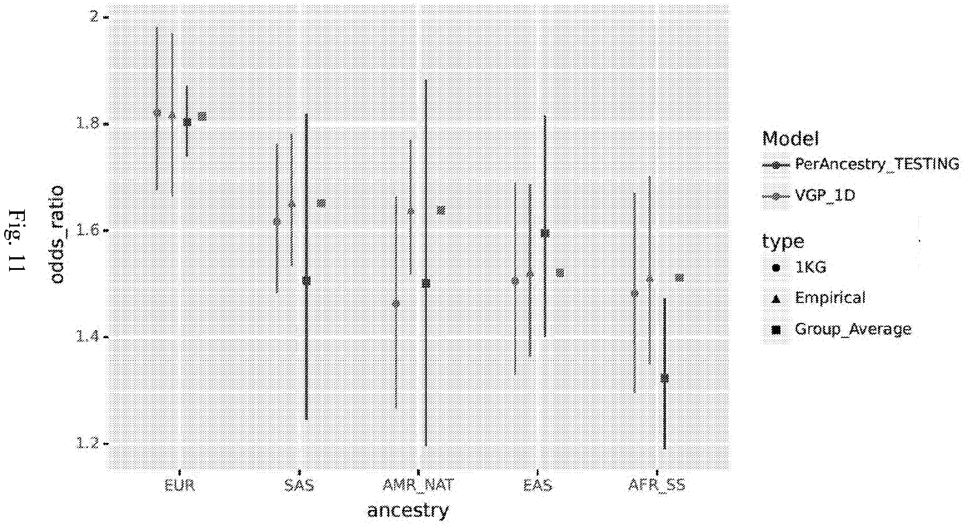

Fig. 11 shows effect size estimates for an individual at the average location of each super-population. The effect size estimates are derived from a GP framework, using model (7), a zero mean function, and the kernel in (8). Effect size estimates are reported for each of the five major super-populations in 1,000 Genomes using a single distance from the European cluster centre (as defined above) and its interaction with PRS in a GP framework. Fitted estimates at the centroids of the super-populations (blue) are compared to the discrete estimate within that population in testing (red). Blue circles ("1KG") refers to the odds ratio at the median PC location of the super-population in 1000 Genomes and blue triangles ("Empirical") refer to the odds ratio at the mean PC location of the testing set individuals within a given super-population. The blue square point estimate ("Group Average") represents the average effect size estimate across individuals within each super-population label.

This GP implementation of the single-dimensional, relative position model allows for non-linear changes in PRS effect sizes as a function of the distance from the reference point, i.e. it incorporates the empirically-observed relationship between heritability explained by PRS and genomic distance from the reference point, but does not constrain this relationship to be linear. As with the other single-dimensional (relative position) ancestry space models considered herein, the model assumes that the change in heritability explained by the PRS is independent of the direction we move from the reference point in the ancestry space. A downside of this is that this assumption can have undue impact on effect size estimates for regions of ancestry space for which training data is available, and can provide overly confident effect size estimates in regions where little training data is available.

**Contributions not dependent on both PRS and position**

All of the example models presented herein are amenable to extension. For example, further PRS trained in distinct or similar ancestries can be incorporated by adding new standardised PRS and interaction terms to the models. The aggregate genetic contribution of these PRS to the risk for the target individual can then be evaluated.

The components of the genetic contribution (or its sub-contributions) dependent on both the PRS and the individual position are generally referred to as interaction terms in the above. In addition, as shown in the various models above, the genetic contribution can include components that are not dependent on both the PRS and the individual position. The genetic contribution may comprise an ancestry-independent component calculated using the polygenic risk score that is not dependent on the individual position. For example, the ancestry-independent component is denoted $\beta_{PRS} x_{PRS,i}$ in model (4) above. Similarly, the genetic contribution may comprise an ancestry-dependent component calculated based on the individual position that is not dependent on the polygenic risk score. For example, the ancestry-dependent component is denoted $\beta_{EUR} x_{EUR,i}$ in model (4) above.

**Other Features**

As mentioned above, a dependency of the genetic contribution on the individual position and the polygenic risk score is determined using training data from a plurality of training individuals having a plurality of different ancestries. The training data comprises, for each of the training individuals, genetic data, and whether the training individual has the target phenotype or phenotype combination. The training data may differ from the reference genetic data used to define the ancestry space.

*Integrated risk tool*

In some embodiments, the training data further comprises, for each of the training individuals, data informative about one or more non-genetic covariates, and the genetic contribution is jointly estimated in the presence of the non-genetic covariates. For example, the non-genetic covariates may include one or more of weight, height, behavioural characteristics, medical traits and other biomarkers, such as blood or urine based measurements.

This allows the model to take account of correlations between genetic and non-genetic covariates when determining the dependency of the genetic contribution, which can further improve accuracy. In such embodiment, the method further comprises receiving individual covariate data for the target individual, the individual covariate data informative about the additional non-genetic covariates for the target individual. Calculating the genetic contribution may further comprise using the individual covariate data. Alternatively or additionally, the individual covariate data may be used in calculating a non-genetic contribution to the risk for the target individual.

## Outputs

Once it has been calculated, the genetic contribution to risk can be output. In some embodiments, the method further comprises outputting the genetic contribution to the risk. Alternatively or additionally, the genetic contribution can be used as part of further calculations that are used to calculate other useful metrics for assessing an individual's risk of developing a particular phenotype or phenotype combination.

In some embodiments, the risk is a relative risk in relation to an individual having an average estimated genetic contribution. This average estimated genetic contribution may be based on average genetic data for similar individuals, for example individuals having similar ancestry. The average estimated genetic contribution may also be based on an average prevalence of the phenotype or phenotype combination in the population, or in similar individuals. In such embodiments, the method further comprises calculating S50 the relative risk for the target individual for the target phenotype or target phenotype combination using the genetic contribution calculated using any of the methods described above of any preceding claim and a non-genetic contribution to the relative risk, and outputting S60 the relative risk. As mentioned above, the non-genetic contribution may be calculated based on non-genetic covariates.

Calculating the relative risk may comprise using a loss function to determine a value of the relative risk for the target individual from a distribution of the relative risk for the target individual. For example, the loss function may be a mean-squared error function or an asymmetric exponential loss function.

As well as relative risk, the method can be used to calculate absolute risk. In some embodiments, the method further comprises calculating an absolute risk for the target individual for the target phenotype or target phenotype combination using the genetic contribution calculated using the method of any preceding claim, and outputting the absolute risk.

## Claims

1. A computer-implemented method of analysing genetic data comprising:

   receiving a polygenic risk score (S10) for a target phenotype or target phenotype combination for a target individual;

   receiving individual genetic data (S20) for the target individual, the individual genetic data (20) informative about an ancestry of the target individual;

   determining an individual position (S30) in an ancestry space using the individual genetic data (20), the individual position represented by a combination of continuous or pseudo-continuous variables, or the individual position comprising an assignment to a weighted combination of a plurality of ancestries; and

   calculating a genetic contribution (S40) to a risk for the target individual for the target phenotype or target phenotype combination using the polygenic risk score (10) and the individual position, the genetic contribution to the risk having a continuous or pseudo-continuous dependency on the individual position,

   wherein the ancestry space is defined using reference genetic data from a plurality of reference individuals having a plurality of different ancestries.

2. The method of claim 1, wherein the genetic contribution comprises a sum of sub-contributions for each axis of the ancestry space, each sub-contribution calculated using a coordinate of the individual position along the respective axis of the ancestry space, optionally wherein

   i) the ancestry space is non-isotropic, such that a dependency of each sub-contribution on the respective coordinate of the individual position differs between the sub-contributions, optionally wherein at least two of the sub-contributions have dependencies on the respective coordinates of the individual position that are related by a shared prior, further optionally wherein the shared prior is specified such that the dependencies of the at least two sub-contributions are sampled from the same distribution, further optionally wherein the shared prior is determined using training data from a plurality of training individuals and one or more predetermined hyperparameters; and/or

ii) each sub-contribution comprises a product of the polygenic risk score and the coordinate of the individual position along the respective axis of the ancestry space.

**3.** The method of claim 1 or 2, wherein calculating the genetic contribution comprises calculating a distance in the ancestry space between the individual position and a reference position in the ancestry space, and calculating the genetic contribution using the distance,

optionally wherein:

i) the reference position is a position in the ancestry space of an ancestry used to train coefficients used to calculate the polygenic risk score; and/or

ii) the ancestry space is defined using reference genetic data from a plurality of reference individuals having a plurality of different ancestries; and

calculating the genetic contribution comprises scaling each axis of the ancestry space using a variance accounted for by the respective axis in the reference genetic data before calculating the distance; and/or

iii) the distance is a Euclidean distance in the ancestry space; and/or

iv) the genetic contribution comprises a product of the polygenic risk score and the distance.

**4.** The method of any preceding claim, wherein calculating the genetic contribution comprises either:

i) using a linear dependency on the individual position; or

ii) using a non-linear dependency on the individual position, optionally wherein the non-linear dependency comprises a regularised function, and/or a penalised B-spline.

**5.** The method of any of claims 1 to 3, wherein calculating the genetic contribution comprises using a non-linear dependency on the individual position, and the non-linear dependency is determined using a Gaussian process as a prior distribution for calculating the genetic contribution using Bayesian inference, optionally wherein:

i) either the Gaussian process has a zero mean function, or the Gaussian process has a mean vector corresponding to a prior estimate of the genetic contribution to the risk for the target individual; and/or

ii) a kernel function of the Gaussian process is a stationary function; and/or

iii) a kernel function of the Gaussian process decays to zero with decreasing similarity between samples; and/or

iv) the kernel function is a radial basis function or a rational quadratic covariance function; and/or

v) a posterior distribution for the Bayesian inference is determined using the Gaussian process and training data from a plurality of training individuals having a plurality of different ancestries, optionally wherein determining the posterior distribution comprises approximating the posterior distribution as a Gaussian distribution.

**6.** The method of claim 5, wherein a kernel function of the Gaussian process depends on one or more hyperparameters, optionally wherein the hyperparameters comprise a hyperparameter associated with each of the polygenic risk score, the individual position, and an interaction between the polygenic risk score and the position.

**7.** The method of any preceding claim, wherein:

i) the genetic contribution comprises an ancestry-independent component calculated using the polygenic risk score that is not dependent on the individual position; and/or

ii) the genetic contribution comprises an ancestry-dependent component calculated based on the individual position that is not dependent on the polygenic risk score; and/or

iii) each reference individual is assigned to one of a plurality of ancestries.

**8.** The method of any preceding claim, wherein a coordinate system of the ancestry space is determined by applying dimension reduction to the reference genetic data, optionally wherein:

i) either the dimension reduction comprises principal component analysis, independent component analysis, non-negative matrix factorisation, or factor analysis, or the dimension reduction comprises a discretisation of the ancestry space into a finite set of ancestries, and the individual position comprises a continuous or pseudo-continuous membership proportion for each ancestry of the finite set of ancestries; and/or

ii) the coordinate system of the ancestry space is chosen to maximise the variance in the reference genetic data accounted for by the ancestry space.

9. The method of any preceding claim, wherein the ancestry space has a dimensionality lower than that of the individual genetic data, and determining the individual position comprises projecting the individual genetic data onto the ancestry space.

10. The method of any preceding claim, wherein a dependency of the genetic contribution on the individual position and the polygenic risk score is determined using training data from a plurality of training individuals having a plurality of different ancestries, the training data comprising, for each of the training individuals, genetic data, and whether the training individual has the target phenotype or phenotype combination,

   optionally wherein: the training data further comprises, for each of the training individuals, data informative about one or more non-genetic covariates, and the genetic contribution is jointly estimated in the presence of the non-genetic covariates; and the method further comprises receiving individual covariate data for the target individual, the individual covariate data informative about the additional non-genetic covariates for the target individual, further optionally wherein the non-genetic covariates include one or more of weight, height, behavioural characteristics, medical traits and other biomarkers, such as blood or urine based measurements.

11. The method of any preceding claim, wherein the risk is a relative risk in relation to an individual having an average estimated genetic contribution, and the method further comprises:

   calculating the relative risk for the target individual for the target phenotype or target phenotype combination using the genetic contribution and a non-genetic contribution to the relative risk; and
   outputting the relative risk and/or the genetic contribution to the risk,
   optionally wherein calculating the relative risk comprises using a loss function to determine a value of the relative risk for the target individual from a distribution of the relative risk for the target individual,
   further optionally wherein the loss function is a mean-squared error function or an asymmetric exponential loss function.

12. The method of any preceding claim, further comprising:

   calculating an absolute risk for the target individual for the target phenotype or target phenotype combination using the genetic contribution; and
   outputting the absolute risk and/or the genetic contribution to the risk.

13. The method of claim 11 or 12, wherein calculating the absolute or relative risk comprises determining a hazard ratio for the target individual, the hazard ratio being normalised using the polygenic risk score and the genetic contribution.

14. A computer program or a computer-readable medium comprising instructions which, when the program is executed by a computer, causes the computer to carry out the method of any of claims 1 to 13.

15. An apparatus for analysing genetic data comprising a processor configured to:

   receive a polygenic risk score for a target phenotype or target phenotype combination for a target individual;
   receive individual genetic data for the target individual, the genetic data informative about an ancestry of the target individual;
   determine an individual position in an ancestry space using the individual genetic data; and
   calculate a genetic contribution to a risk for the target individual for the target phenotype or target phenotype combination using the polygenic risk score and the individual position, wherein:

      the individual position is represented by a combination of continuous or pseudo-continuous variables, or the individual position comprises an assignment to a weighted combination of a plurality of ancestries;
      the ancestry space is defined using reference genetic data from a plurality of reference individuals having a plurality of different ancestries; and
      the genetic contribution has a continuous or pseudo-continuous dependency on the individual position.

**Patentansprüche**

1. Computer-implementiertes Verfahren zur Analyse genetischer Daten, das Folgendes umfasst:

   Empfangen eines polygenen Risikowerts (S 10) für einen Zielphänotyp oder eine Zielphänotypkombination für ein Zielindividuum;

   Empfangen individueller genetischer Daten (S20) für das Zielindividuum, wobei die individuellen genetischen Daten (20) Informationen über eine Abstammung des Zielindividuums enthalten;

   Bestimmen einer individuellen Position (S30) in einem Abstammungsraum unter Verwendung der individuellen genetischen Daten (20), wobei die individuelle Position durch eine Kombination von kontinuierlichen oder pseudokontinuierlichen Variablen dargestellt wird, oder die individuelle Position eine Zuordnung zu einer gewichteten Kombination einer Vielzahl von Abstammungen umfasst; und

   Berechnen eines genetischen Beitrags (S40) zu einem Risiko für das Zielindividuum für den Zielphänotyp oder die Zielphänotypkombination unter Verwendung des polygenen Risikowerts (10) und der individuellen Position, wobei der genetische Beitrag zu dem Risiko eine kontinuierliche oder pseudokontinuierliche Abhängigkeit von der individuellen Position hat,

   wobei der Abstammungsraum unter Verwendung genetischer Referenzdaten von einer Vielzahl von Referenzindividuen mit einer Vielzahl von unterschiedlichen Abstammungen definiert wird.

2. Verfahren nach Anspruch 1, wobei der genetische Beitrag eine Summe von Teilbeiträgen für jede Achse des Abstammungsraums umfasst, wobei jeder Teilbeitrag unter Verwendung einer Koordinate der individuellen Position entlang der jeweiligen Achse des Abstammungsraums berechnet wird, optional wobei

   i) der Abstammungsraum nicht isotrop ist, so dass eine Abhängigkeit jedes Teilbeitrags von der jeweiligen Koordinate der individuellen Position zwischen den Teilbeiträgen unterschiedlich ist, optional wobei mindestens zwei der Teilbeiträge Abhängigkeiten von den jeweiligen Koordinaten der individuellen Position aufweisen, die durch einen gemeinsamen Prior in Beziehung stehen, ferner optional wobei der gemeinsame Prior so spezifiziert ist, dass die Abhängigkeiten der mindestens zwei Teilbeiträge aus der gleichen Verteilung gesampelt werden, ferner optional wobei der gemeinsame Prior unter Verwendung von Trainingsdaten von einer Vielzahl von Trainingsindividuen und einem oder mehreren vorbestimmten Hyperparametern bestimmt wird; und/oder

   ii) jeder Teilbeitrag ein Produkt aus dem polygenen Risikowert und der Koordinate der individuellen Position entlang der jeweiligen Achse des Abstammungsraums umfasst.

3. Verfahren nach Anspruch 1 oder 2, wobei das Berechnen des genetischen Beitrags das Berechnen eines Abstands in dem Abstammungsraum zwischen der individuellen Position und einer Referenzposition in dem Abstammungsraum und das Berechnen des genetischen Beitrags unter Verwendung des Abstands umfasst, optional wobei:

   i) die Referenzposition eine Position im Abstammungsraum einer Abstammung ist, die verwendet wird, um Koeffizienten zu trainieren, die zur Berechnung des polygenen Risikowerts verwendet werden; und/oder

   ii) der Abstammungsraum unter Verwendung genetischer Referenzdaten von einer Vielzahl von Referenzindividuen mit einer Vielzahl von unterschiedlichen Abstammungen definiert wird; und

   die Berechnung des genetischen Beitrags die Skalierung jeder Achse des Abstammungsraums unter Verwendung einer Varianz umfasst, die durch die jeweilige Achse in den genetischen Referenzdaten berücksichtigt wird, bevor der Abstand berechnet wird; und/oder

   iii) der Abstand ein euklidischer Abstand im Abstammungsraum ist; und/oder

   iv) der genetische Beitrag ein Produkt aus dem polygenen Risikowert und dem Abstand ist.

4. Verfahren nach einem der vorhergehenden Ansprüche, wobei das Berechnen des genetischen Beitrags Folgendes umfasst:

   i) die Verwendung einer linearen Abhängigkeit von der individuellen Position; oder

   ii) die Verwendung einer nicht-linearen Abhängigkeit von der individuellen Position, wobei die nicht-lineare Abhängigkeit optional eine regularisierte Funktion und/oder einen bestraften B-Spline umfasst.

5. Verfahren nach einem der Ansprüche 1 bis 3, wobei die Berechnung des genetischen Beitrags die Verwendung einer nicht-linearen Abhängigkeit von der individuellen Position umfasst und die nicht-lineare Abhängigkeit unter Verwendung eines Gauß-Prozesses als Prior-Verteilung für die Berechnung des genetischen Beitrags unter Verwendung von Bayes'scher Inferenz bestimmt wird, optional wobei:

i) entweder der Gauß-Prozess eine Null-Mittelwert-Funktion hat, oder der Gauß-Prozess einen Mittelwertvektor hat, der einer Vorabschätzung des genetischen Beitrags zum Risiko für das Zielindividuum entspricht; und/oder
ii) eine Kernel-Funktion des Gauß-Prozesses eine stationäre Funktion ist; und/oder
iii) eine Kernfunktion des Gauß-Prozesses mit abnehmender Ähnlichkeit zwischen den Proben auf Null abnimmt; und/oder
iv) die Kernfunktion eine radiale Basisfunktion oder eine rationale quadratische Kovarianzfunktion ist; und/oder
v) eine posteriore Verteilung für die Bayes'sche Inferenz unter Verwendung des Gauß-Prozesses und von Trainingsdaten aus einer Vielzahl von Trainingsindividuen mit einer Vielzahl von unterschiedlichen Abstammungen bestimmt wird, optional wobei das Bestimmen der posterioren Verteilung das Approximieren der posterioren Verteilung als eine Gauß-Verteilung umfasst.

6. Verfahren nach Anspruch 5, wobei eine Kernel-Funktion des Gauß-Prozesses von einem oder mehreren Hyperparametern abhängt, optional wobei die Hyperparameter einen Hyperparameter umfassen, der mit jedem von dem polygenen Risikowert, der individuellen Position und einer Interaktion zwischen dem polygenen Risikowert und der Position verbunden ist.

7. Verfahren nach einem der vorhergehenden Ansprüche, wobei:

i) der genetische Beitrag eine abstammungsunabhängige Komponente umfasst, die unter Verwendung des polygenen Risikowerts berechnet wird, der nicht von der individuellen Position abhängt; und/oder
ii) der genetische Beitrag eine auf der Basis der individuellen Position berechnete abstammungsabhängige Komponente umfasst, die nicht von dem polygenen Risikowert abhängig ist; und/oder
iii) jedes Referenzindividuum einer von mehreren Abstammungen zugeordnet wird.

8. Verfahren nach einem der vorhergehenden Ansprüche, wobei ein Koordinatensystem des Abstammungsraumes durch Anwendung einer Dimensionsreduktion auf die genetischen Referenzdaten bestimmt wird, wobei optional:

i) entweder die Dimensionsreduktion eine Hauptkomponentenanalyse, eine unabhängige Komponentenanalyse, eine nichtnegative Matrixfaktorisierung oder eine Faktorenanalyse umfasst, oder die Dimensionsreduktion eine Diskretisierung des Abstammungsraums in einen endlichen Satz von Abstammungen umfasst, und die individuelle Position einen kontinuierlichen oder pseudokontinuierlichen Zugehörigkeitsanteil für jede Abstammung des endlichen Satzes von Abstammungen umfasst; und/oder
ii) das Koordinatensystem des Abstammungsraums so gewählt wird, dass die Varianz in den genetischen Referenzdaten, die durch den Abstammungsraum berücksichtigt wird, maximiert wird.

9. Verfahren nach einem der vorhergehenden Ansprüche, wobei der Abstammungsraum eine geringere Dimensionalität als die der individuellen genetischen Daten aufweist und die Bestimmung der individuellen Position das Projizieren der individuellen genetischen Daten auf den Abstammungsraum umfasst.

10. Verfahren nach einem der vorhergehenden Ansprüche, wobei eine Abhängigkeit des genetischen Beitrags von der individuellen Position und dem polygenen Risikowert unter Verwendung von Trainingsdaten von einer Vielzahl von Trainingsindividuen mit einer Vielzahl von unterschiedlichen Abstammungen bestimmt wird, wobei die Trainingsdaten für jedes der Trainingsindividuen genetische Daten und die Angabe umfassen, ob das Trainingsindividuum den Zielphänotyp oder die Zielphänotypkombination aufweist,

optional wobei: die Trainingsdaten für jedes der Trainingsindividuen ferner Daten umfassen, die über eine oder mehrere nicht-genetische Kovariaten informativ sind, und der genetische Beitrag in Gegenwart der nicht-genetischen Kovariaten gemeinsam geschätzt wird; und das Verfahren ferner das Empfangen individueller Kovariatendaten für das Zielindividuum umfasst, wobei die individuellen Kovariatendaten über die zusätzlichen nicht-genetischen Kovariaten für das Zielindividuum informativ sind,
wobei die nicht-genetischen Kovariaten ferner optional eines oder mehrere der Folgenden umfassen: Gewicht, Größe, Verhaltensmerkmale, medizinische Eigenschaften und andere Biomarker, wie z.B. auf Blut oder Urin basierende Messungen.

11. Verfahren nach einem der vorhergehenden Ansprüche, wobei das Risiko ein relatives Risiko in Bezug auf ein Individuum mit einem durchschnittlichen geschätzten genetischen Beitrag ist, und das Verfahren ferner Folgendes umfasst:

Berechnen des relativen Risikos für das Zielindividuum für den Zielphänotyp oder die Zielphänotypkombination unter Verwendung des genetischen Beitrags und eines nicht-genetischen Beitrags zu dem relativen Risiko; und Ausgeben des relativen Risikos und/oder des genetischen Beitrags zu dem Risiko, wobei die Berechnung des relativen Risikos optional die Verwendung einer Verlustfunktion umfasst, um einen Wert des relativen Risikos für das Zielindividuum aus einer Verteilung des relativen Risikos für das Zielindividuum zu bestimmen, wobei die Verlustfunktion optional eine Funktion des mittleren quadratischen Fehlers oder eine asymmetrische exponentielle Verlustfunktion ist.

12. Verfahren nach einem der vorhergehenden Ansprüche, das ferner Folgendes umfasst:

Berechnen eines absoluten Risikos für das Zielindividuum für den Zielphänotyp oder die Zielphänotypkombination unter Verwendung des genetischen Beitrags; und Ausgeben des absoluten Risikos und/oder des genetischen Beitrags zu dem Risiko.

13. Verfahren nach Anspruch 11 oder 12, wobei die Berechnung des absoluten oder relativen Risikos die Bestimmung eines Gefährdungsverhältnisses für das Zielindividuum umfasst, wobei das Gefährdungsverhältnis unter Verwendung des polygenen Risikowerts und des genetischen Beitrags normiert wird.

14. Computerprogramm oder computerlesbares Medium, das Anweisungen enthält, die, wenn das Programm von einem Computer ausgeführt wird, den Computer veranlassen, das Verfahren nach einem der Ansprüche 1 bis 13 durchzuführen.

15. Vorrichtung zum Analysieren genetischer Daten, die einen Prozessor umfasst, der für Folgendes konfiguriert ist:

Empfangen eines polygenen Risikowerts für einen Zielphänotyp oder eine Zielphänotypkombination für ein Zielindividuum; Empfangen individueller genetischer Daten für das Zielindividuum, wobei die genetischen Daten Informationen über eine Abstammung des Zielindividuums enthalten; Bestimmen einer individuellen Position in einem Abstammungsraum unter Verwendung der individuellen genetischen Daten; und Berechnen eines genetischen Beitrags zu einem Risiko für das Zielindividuum für den Zielphänotyp oder die Zielphänotypkombination unter Verwendung des polygenen Risikowerts und der individuellen Position, wobei:

die individuelle Position durch eine Kombination von kontinuierlichen oder pseudokontinuierlichen Variablen dargestellt wird, oder die individuelle Position eine Zuordnung zu einer gewichteten Kombination einer Vielzahl von Abstammungen umfasst; der Abstammungsraum unter Verwendung genetischer Referenzdaten von einer Vielzahl von Referenzindividuen mit einer Vielzahl von unterschiedlichen Abstammungen definiert wird; und der genetische Beitrag eine kontinuierliche oder pseudokontinuierliche Abhängigkeit von der individuellen Position hat.

**Revendications**

1. Procédé d'analyse de données génétiques mis en oeuvre par ordinateur comprenant les étapes consistant à :

recevoir un score de risque polygénique (S10) pour un phénotype cible ou une combinaison de phénotypes cibles pour un individu cible ; recevoir des données génétiques individuelles (S20) pour l'individu cible, les données génétiques individuelles (20) informant sur une ascendance de l'individu cible ; déterminer une position individuelle (S30) dans un espace d'ascendance à l'aide des données génétiques individuelles (20), la position individuelle représentée par une combinaison de variables continues ou pseudo-continues, ou la position individuelle comprenant une affectation à une combinaison pondérée d'une pluralité d'ascendances; et calculer une contribution génétique (S40) à un risque pour l'individu cible pour le phénotype cible ou la combinaison de phénotypes cibles à l'aide du score de risque polygénique (10) et de la position individuelle, la contribution génétique au risque ayant une dépendance continue ou pseudo-continue vis-à-vis de la position individuelle,

dans lequel l'espace d'ascendance est défini à l'aide de données génétiques de référence provenant d'une pluralité d'individus de référence ayant une pluralité d'ascendances différentes.

2. Procédé selon la revendication 1, dans lequel la contribution génétique comprend une somme de sous-contributions pour chaque axe de l'espace d'ascendance, chaque sous-contribution étant calculée en utilisant une coordonnée de la position individuelle le long de l'axe respectif de l'espace d'ascendance, éventuellement dans lequel :

i) l'espace d'ascendance est non isotrope, de sorte qu'une dépendance de chaque sous-contribution sur la coordonnée respective de la position individuelle diffère entre les sous-contributions, éventuellement dans lequel au moins deux des sous-contributions ont des dépendances sur les coordonnées respectives de la position individuelle qui sont liées par une distribution préalable partagée, en outre éventuellement dans lequel la distribution préalable partagée est spécifiée de sorte que les dépendances des au moins deux sous-contributions sont échantillonnées à partir de la même distribution, en outre éventuellement dans lequel la distribution préalable partagée est déterminée à l'aide de données d'apprentissage à partir d'une pluralité d'individus d'apprentissage et d'un ou plusieurs hyperparamètres prédéterminés ; et/ou

ii) chaque sous-contribution comprend un produit du score de risque polygénique et de la coordonnée de la position individuelle le long de l'axe respectif de l'espace d'ascendance.

3. Procédé selon la revendication 1 ou 2, dans lequel le calcul de la contribution génétique comprend le calcul d'une distance dans l'espace d'ascendance entre la position individuelle et une position de référence dans l'espace d'ascendance, et le calcul de la contribution génétique en utilisant la distance, éventuellement dans lequel :

i) la position de référence est une position dans l'espace d'ascendance d'une ascendance utilisée pour former des coefficients utilisés pour calculer le score de risque polygénique ; et/ou

ii) l'espace d'ascendance est défini à l'aide de données génétiques de référence provenant d'une pluralité d'individus de référence ayant une pluralité d'ascendances différentes ; et

le calcul de la contribution génétique comprend la mise à l'échelle de chaque axe de l'espace d'ascendance à l'aide d'une variance prise en compte par l'axe respectif dans les données génétiques de référence avant le calcul de la distance ; et/ou

iii) la distance est une distance euclidienne dans l'espace d'ascendance ; et/ou

iv) la contribution génétique comprend un produit du score de risque polygénique et de la distance.

4. Procédé selon l'une quelconque des revendications précédentes, dans lequel le calcul de la contribution génétique comprend soit :

i) l'utilisation d'une dépendance linéaire à la position individuelle ; ou

ii) l'utilisation d'une dépendance non linéaire à la position individuelle, éventuellement dans lequel la dépendance non linéaire comprend une fonction régularisée, et/ou une B-spline pénalisée.

5. Procédé selon l'une quelconque des revendications 1 à 3, dans lequel le calcul de la contribution génétique comprend l'utilisation d'une dépendance non linéaire à la position individuelle, et la dépendance non linéaire est déterminée à l'aide d'un processus gaussien en tant que distribution antérieure pour calculer la contribution génétique à l'aide d'une inférence bayésienne, éventuellement dans lequel :

i) soit le processus gaussien a une fonction moyenne nulle, soit le processus gaussien a un vecteur moyen correspondant à une estimation préalable de la contribution génétique au risque pour l'individu cible ; et/ou

ii) une fonction noyau du processus gaussien est une fonction stationnaire ; et/ou

iii) une fonction noyau du processus gaussien se désintègre à zéro avec une similarité décroissante entre les échantillons ; et/ou

iv) la fonction noyau est une fonction de base radiale ou une fonction de covariance quadratique rationnelle ; et/ou

v) une distribution postérieure pour l'inférence bayésienne est déterminée à l'aide du processus gaussien et des données d'apprentissage d'une pluralité d'individus d'apprentissage ayant une pluralité d'ascendances différentes, éventuellement dans lequel la détermination de la distribution postérieure comprend l'approximation de la distribution postérieure comme une distribution gaussienne.

**6.** Procédé selon la revendication 5, dans lequel une fonction noyau du processus gaussien dépend d'un ou plusieurs hyperparamètres, éventuellement dans lequel les hyperparamètres comprennent un hyperparamètre associé à chacun parmi le score de risque polygénique, la position individuelle, et une interaction entre le score de risque polygénique et la position.

**7.** Procédé selon l'une quelconque des revendications précédentes, dans lequel :

i) la contribution génétique comprend une composante indépendante de l'ascendance calculée à l'aide du score de risque polygénique qui ne dépend pas de la position individuelle ; et/ou
ii) la contribution génétique comprend une composante dépendante de l'ascendance calculée en fonction de la position individuelle qui ne dépend pas du score de risque polygénique ; et/ou
iii) chaque individu de référence est attribué à l'une d'une pluralité d'ascendances.

**8.** Procédé selon l'une quelconque des revendications précédentes, dans lequel un système de coordonnées de l'espace d'ascendance est déterminé en appliquant une réduction de dimension aux données génétiques de référence, éventuellement dans lequel :

i) soit la réduction de dimension comprend une analyse en composantes principales, une analyse en composantes indépendantes, une factorisation matricielle non négative ou une analyse factorielle, soit la réduction de dimension comprend une discrétisation de l'espace d'ascendance en un ensemble fini d'ascendances, et la position individuelle comprend une proportion d'appartenance continu ou pseudo-continue pour chaque ascendance de l'ensemble fini d'ascendances ; et/ou
ii) le système de coordonnées de l'espace d'ascendance est choisi pour maximiser la variance des données génétiques de référence représentée par l'espace d'ascendance.

**9.** Procédé selon l'une quelconque des revendications précédentes, dans lequel l'espace d'ascendance a une dimensionnalité inférieure à celle des données génétiques individuelles, et la détermination de la position individuelle comprend la projection des données génétiques individuelles sur l'espace d'ascendance.

**10.** Procédé selon l'une quelconque des revendications précédentes, dans lequel une dépendance de la contribution génétique à la position individuelle et au score de risque polygénique est déterminée à l'aide de données d'apprentissage provenant d'une pluralité d'individus d'apprentissage ayant une pluralité d'ascendances différentes, les données d'apprentissage comprenant, pour chacun des individus d'apprentissage, les données génétiques, et si l'individu d'apprentissage a le phénotype cible ou la combinaison de phénotypes, éventuellement dans lequel : les données d'apprentissage comprennent en outre, pour chacun des individus d'apprentissage, des données informatives sur une ou plusieurs covariables non génétiques, et la contribution génétique est estimée conjointement en présence des covariables non génétiques ; et le procédé comprend en outre la réception de données de covariables individuelles pour l'individu cible, les données de covariables individuelles informant sur les covariables non génétiques supplémentaires pour l'individu cible,
en outre éventuellement dans lequel les covariables non génétiques comprennent un ou plusieurs parmi le poids, la taille, les caractéristiques comportementales, les traits médicaux et d'autres biomarqueurs, tels que les mesures basées sur le sang ou l'urine.

**11.** Procédé selon l'une quelconque des revendications précédentes, dans lequel le risque est un risque relatif par rapport à un individu ayant une contribution génétique estimée moyenne, et le procédé comprend en outre les étapes consistant à :

calculer le risque relatif pour l'individu cible pour le phénotype cible ou la combinaison de phénotypes cibles en utilisant la contribution génétique et une contribution non génétique au risque relatif ; et
sortir le risque relatif et/ou la contribution génétique au risque,
éventuellement dans lequel le calcul du risque relatif comprend l'utilisation d'une fonction de perte pour déterminer une valeur du risque relatif pour l'individu cible à partir d'une distribution du risque relatif pour l'individu cible, en outre éventuellement dans lequel la fonction de perte est une fonction d'erreur quadratique moyenne ou une fonction de perte exponentielle asymétrique.

**12.** Procédé selon l'une quelconque des revendications précédentes, comprenant en outre les étapes consistant à :

calculer un risque absolu pour l'individu cible pour le phénotype cible ou la combinaison de phénotypes cibles en utilisant la contribution génétique ; et

sortir le risque absolu et/ou la contribution génétique au risque.

13. Procédé selon la revendication 11 ou 12, dans lequel le calcul du risque absolu ou relatif comprend la détermination d'un rapport de risque pour l'individu cible, le rapport de risque étant normalisé en utilisant le score de risque polygénique et la contribution génétique.

14. Programme informatique ou support lisible par ordinateur comprenant des instructions qui, lorsque le programme est exécuté par un ordinateur, amènent l'ordinateur à exécuter le procédé selon l'une quelconque des revendications 1 à 13.

15. Appareil pour analyser des données génétiques comprenant un processeur configuré pour :

recevoir un score de risque polygénique pour un phénotype cible ou une combinaison de phénotypes cibles pour un individu cible ;

recevoir des données génétiques individuelles pour l'individu cible, les données génétiques informant sur une ascendance de l'individu cible ;

déterminer une position individuelle dans un espace d'ascendance à l'aide des données génétiques individuelles ; et

calculer une contribution génétique à un risque pour l'individu cible pour le phénotype cible ou

la combinaison de phénotypes cibles en utilisant le score de risque polygénique et la position individuelle, dans lequel :

la position individuelle est représentée par une combinaison de variables continues ou pseudo-continues, ou la position individuelle comprend une affectation à une combinaison pondérée d'une pluralité d'ascendances ;

l'espace d'ascendance est défini à l'aide de données génétiques de référence provenant d'une pluralité d'individus de référence ayant une pluralité d'ascendances différentes ; et

la contribution génétique a une dépendance continue ou pseudo-continue à la position individuelle.

Fig. 1

PRS
10

→

Receive PRS
S10

↓

Individual
genetic data
20

→

Receive individual
data
S20

↓

Determine
individual position
S30

↓

Calculate genetic
contribution
S40

↓

Non-genetic
contribution
30

→

Calculate relative
risk
S50

↓

Relative risk
40

←

Output relative risk
S60

Fig. 2

Fig. 3

Fig. 4

Fig. 5

Fig. 6

Fig. 7

Fig. 8

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MINXIAN WANG et al.** Validation of a Genome-Wide Polygenic Score for Coronary Artery Disease in South Asians. *Journal of the American College of Cardiology,* 03 August 2020, vol. 76 (6), 703-714

- **AMARIUTA, T. ; ISHIGAKI, K. ; SUGISHITA, H. et al.** *Methods,* 2020
- **FRITSCHE et al.** *Methods,* 2021